# EUROPÄISCHE PATENTSCHRIFT

(11) **EP 0 754 668 B1**
(45) Veröffentlichungstag und Bekanntmachung des Hinweises auf die Patenterteilung: **05.02.2003**
(21) Anmeldenummer: 96111179.6
(22) Anmeldetag: 11.07.1996
(51) Int. Cl.: C07C 43/174, C07C 43/176, C09K 19/12, C09K 19/30, C09K 19/34, C09K 19/42

(54) **Fluorbenzylether-Derivate**
Fluorobenzylether derivatives
Dérivés de l'éther fluoro-benzylique

(30) Priorität: 17.07.1995 CH 209695
(43) Veröffentlichungstag der Anmeldung: 22.01.1997
(73) Patentinhaber: Rolic AG, 6301 Zug (CH)
(72) Erfinder: Buchecker, Richard, 8008 Zurich (CH); Germann, Alfred, 4058 Base (CH)
(74) Vertreter: Liebetanz, Michael

(56) Entgegenhaltungen:
- EP-A- 0 543 244
- DE-A- 4 222 371
- DE-A- 4 425 642
- DATABASE WPI Section Ch, Week 9437 Derwent Publications Ltd., London, GB; Class E14, AN 94-299728 XP002032923 & JP 06 228 037 A (KANTO KAGAKU KK) , 16.August 1994
- DATABASE WPI Section Ch, Week 9607 Derwent Publications Ltd., London, GB; Class E14, AN 96-068801 XP002032924 & WO 96 00204 A (CITIZEN WATCH CO LTD) , 4.Januar 1996

## Beschreibung

Die vorliegende Erfindung betrifft neue flüssigkristalline Fluorbenzylether-Derivate, flüssigkristalline Mischungen, welche solche Verbindungen enthalten, sowie die Verwendung solcher Verbindungen und Mischungen in elektro-optischen Vorrichtungen.

Flüssige Kristalle werden vor allem als Dielektrika in Anzeigevorrichtungen verwendet, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super twisted nematic"), SBE-Zellen ("super birefringence effect") und OMI-Zellen ("optical mode interference"). Für Displays mit hohem Informationsgehalt sind in letzter Zeit neben den passiv angesteuerten, multiplexierten Zellen, besonders die aktiv angesteuerten, z.B. TFT-Zellen ("thin film transistor") wichtig geworden. Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische, photochemische und thermische Stabilität und eine gute Stabilität gegenüber elektrischen Feldern besitzen. Ferner sollten sie über einen möglichst breiten Bereich eine geeignete Mesophase aufweisen (beispielsweise für die oben genannten Zellen eine nematische bzw. eine cholesterische Phase), aber trotzdem ausreichend niedere Viskosität besitzen und in den Zellen kurze Ansprechzeiten, tiefe Schwellenspannungen und einen hohen Kontrast ermöglichen. Weitere Eigenschaften, wie die elektrische Leitfähigkeit, die dielektrische Anisotropie und die optische Anisotropie, müssen je nach Anwendungsgebiet und Zellentyp unterschiedlichen Anforderungen genügen. Beispielsweise sollten Materialien für Zellen mit verdrillt nematischer Struktur eine möglichst hohe positive dielektrische Anisotropie und gleichzeitig eine möglichst geringe Leitfähigkeit aufweisen. Diese letztere Eigenschaft ist vorallem für TFT-Zellen von besonderer Wichtigkeit. Leider führen aber Komponenten mit höherer dielektrischer Anisotropie infolge ihres besseren Lösungsvermögens für ionische Verunreinigungen meist zu einer erhöhten Leitfähigkeit in Mischungen. Es werden daher Komponenten gesucht, die sich durch eine möglichst hohe dielektrische Anisotropie bei gleichzeitig möglichst geringer Leitfähigkeit auszeichnen.

Die EP-A-0 543 244 von Hoffmann La Roche offenbart fluorsubstituierte Tolanverbindungen, welche mittels der in der folgenden Beschreibung angegebenen Strukturformel I definiert werden können. Die Eigenschaften dieser Verbindungen hinsichtlich dielektrischer Anisotropie und Leitfähigkeit überzeugen nicht. Dabei weisen die in der EP-A-0 543 244 offenbarten Verbindungen für Z¹ eine C-Dreifachbindung auf.

Die DE 42 22 371 A offenbart ebenfalls Verbindungen, welche mittels der in der folgenden Beschreibung angegebenen Strukturformel I definiert werden können. Dabei werden auf den Seiten 37 und 38 eine Reihe von Verbindungen genannt, welche als Rest R¹ eine fluoroder chlorsubstituierte Alkylgruppe besitzen.

Die DE 44 25 642 A offenbart schliesslich auch Verbindungen, welche mittels der in der folgenden Beschreibung angegebenen Strukturformel I definiert werden können. Dabei besitzen die in dieser Schrift genannten Verbindungen sowohl für R¹ als auch für R² je eine unsubstituierte Alkylgruppe.

Demgegenüber weisen die erfindungsgemässen Verbindungen, welche sich durch tiefe Schmelzpunkte und vergleichsweise hohe Klärpunkte auszeichnen, trotz niedriger Polarität eine vergleichsweise tiefe Schwellenspannung (V₁₀) auf und sind daher insbesondere für die Anwendung in TFT-Zellen geeignet.

Es sind dies Verbindungen der allgemeinen Formel 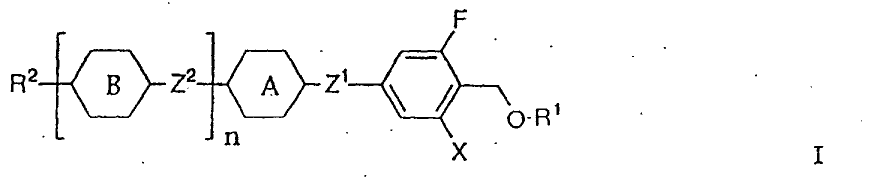 worin
- R¹: C₁-C₅-Alkyl oder C₃-C₅-Alkenyl, worin auch eine CH₂-Gruppe durch ein Sauerstoffatom ersetzt sein kann;
- X: H oder F;
- A und B: unabhängig voneinander trans-1,4-Cyclohexylen, trans-1,3-Dioxan-2,5-diyl, Pyridin-2,5-diyl, Pyrimidin-2,5-diyl oder gegebenenfalls mit 1 oder 2 Fluoratomen substituiertes 1,4-Phenylen;
- Z¹: eine Einfachbindung oder -CH₂-CH₂-
- Z²: unabhängig von Z eine Einfachbindung, -CH₂-CH₂-, -O-CH₂-, -CH₂-O-, -C≡C-, -(CH₂)₄-, -O(CH₂)₃-, -(CH₂)₃O-, -CH=CH-CH₂O- oder -CH=CH- (CH₂)₂-, -OCH₂-CH=CH- oder -(CH₂)₂-CH=CH-; mit der Massgabe, dass mindestens eine der Gruppen Z¹ oder Z² eine Einfachbindung ist;
- n: 0 oder 1; und
- R²: C₂-C₁₂-Alkyl oder C₂-C₁₂-Alkenyl, worin auch eine oder mehrere nicht benachbarte CH₂-Gruppen durch Sauerstoff ersetzt und/oder eines oder mehrere Wasserstoffatome durch Fluoratome ersetzt sein können,
bedeuten, wobei R² dann nicht ein C₂-C₁₂ Alkyl darstellen darf, wenn R¹ ein C₁-C₅ Alkyl ist.

Die in den Definitionen verwendeten Ausdrücke werden im folgenden erläutert:
"C₁-C₅-Alkyl oder C₃-C₅-Alkenyl, worin auch eine CH₂-Gruppe durch ein Sauerstoffatom ersetzt sein kann" umfasst geradkettige Alkylgruppen, wie Methyl, Ethyl, Propyl, Butyl, Pentyl; geradkettige Alkoxyalkylgruppen, wie Methoxymethyl, Methoxyethyl; geradkettige Alkenylgruppen, wie 3E-Pentenyl, 2-Propenyl, 3-Butenyl, 4-Pentenyl; Alkenyloxyalkylgruppen und dergleichen.
"C₂-C₁₂-Alkyl oder C₂-C₁₂-Alkenyl, worin auch eine oder mehrere nicht benachbarte CH₂-Gruppen durch Sauerstoff ersetzt und/oder eines oder mehrere Wasserstoffatome durch Fluoratome ersetzt sein können" umfasst im Rahmen der vorliegenden Erfindung geradkettige Alkylgruppen wie Methyl, Ethyl, Propyl, Butyl, Pentyl, Hexyl, Heptyl, Octyl, Nonyl, Decyl, Undecyl, Dodecyl; Alkoxygruppen, worin der Alkylrest wie oben definiert ist; Alkoxyalkylgruppen, wie beispielsweise Methoxymethyl, Ethoxymethyl, Propyloxymethyl, Methoxyethyl, Ethoxyethyl, Propoxyethyl; 1-Alkenylgruppen, wie 1E-Propenyl, 1E-Butenyl, 1E-Pentenyl, 1E-Hexenyl, 1E-Heptenyl; 3-Alkenylgruppen, wie 3E-Pentenyl, 3E-Hexenyl, 3E-Heptenyl, Alkenylgruppen mit endständiger Doppelbindung, wie Vinyl, 2-Propenyl, 3-Butenyl, 4-Pentenyl, 6-Hexenyl; Alkenyloxygruppen, wie 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy, 7-Octenyloxy, 8-Nonenyloxy, 9-Decenyloxy, 10-Undecenyloxy, 11-Dodecenyloxy; fluorierte Gruppen, wie Fluormethyl, Difluormethyl, Trifluormethyl, 1-Fluorpropyl, 1-Fluorpentyl, 1-Chlorpropyl, 2-Fluorpropyl, 2-Fluoropentyl, 2-Chlorpropyl, 2-Fluorpropyloxy, 2-Fluorbutyloxy, 2-Fluorpentyloxy, 2-Fluorhexyloxy, und dergleichen.

Besonders bevorzugte Verbindungen der Formel I sind diejenigen, worin der Rest R¹ 1 bis 3 Kohlenstoffatome hat und Methyl, Ethyl oder Propyl, insbesondere Methyl, bedeutet. Der Rest R² hat vorzugsweise 2 bis 6 Kohlenstoffatome und bedeutet Ethyl, Propyl, Butyl, Pentyl, Hexyl, Vinyl, 1E-Propenyl, 1E-Butenyl, 1E-Pentenyl, 1E-Hexenyl, 3-Butenyl oder 4-Pentenyl. Z¹ und Z² bedeuten vorzugsweise eine Einfachbindung oder -CH₂CH₂-; und die Ringe A und B bedeuten vorzugsweise trans-1,4-Cyclohexylen, trans-1,3-Dioxan-2,5-diyl oder 1,4-Phenylen.

Besonders bevorzugte Verbindungen der Formel I sind somit die Verbindungen der allgemeinen Formeln 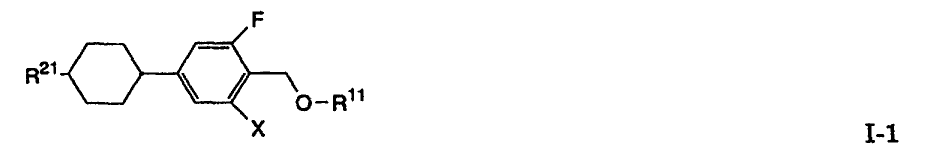 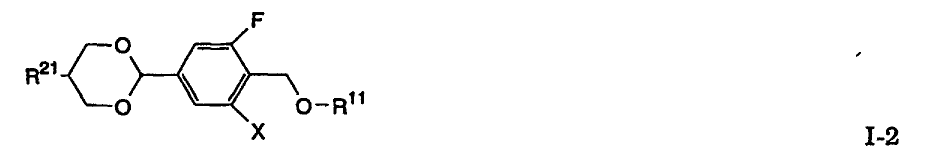 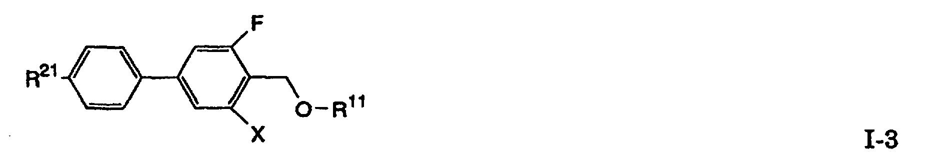 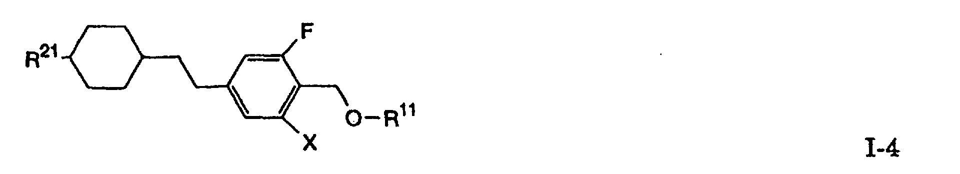 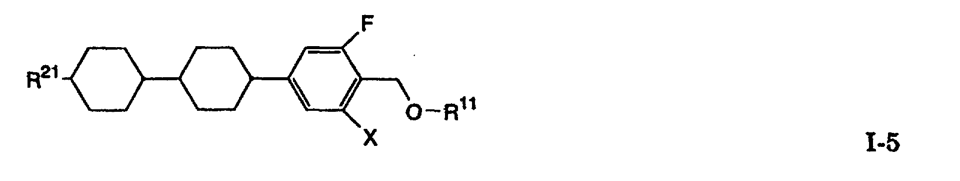 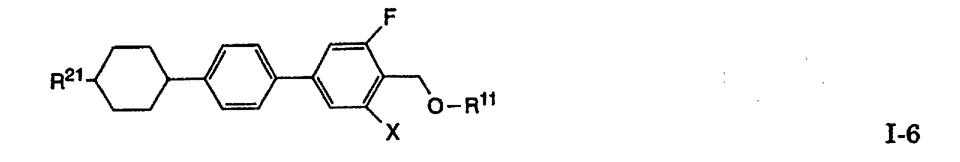 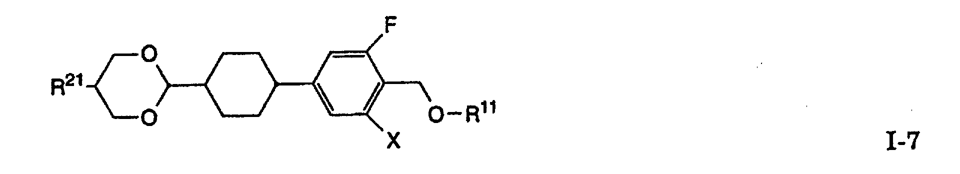 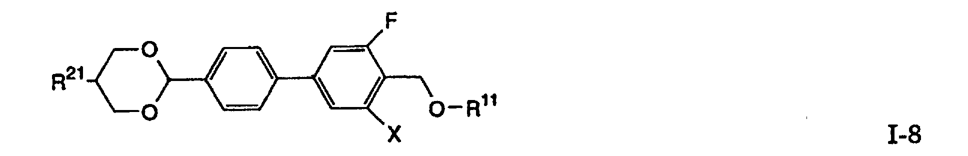 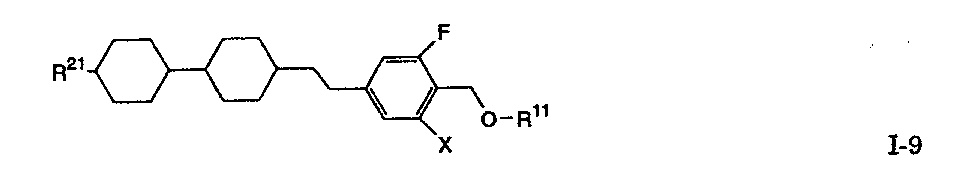 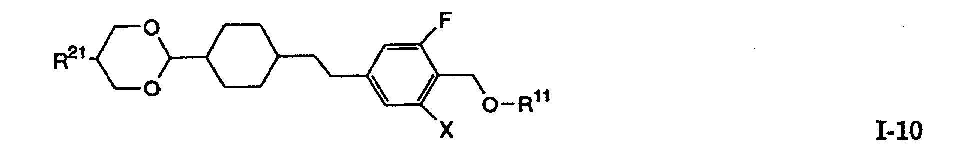 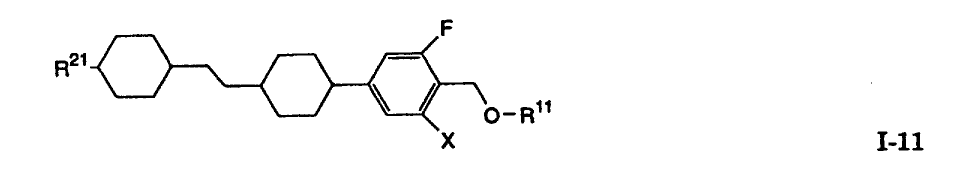 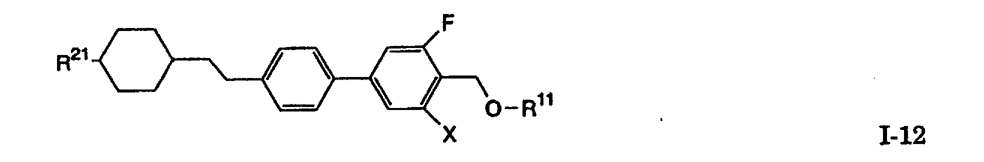 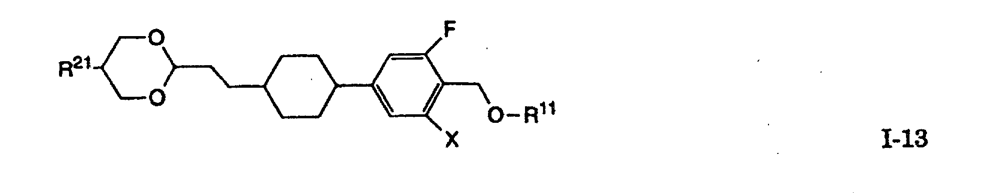 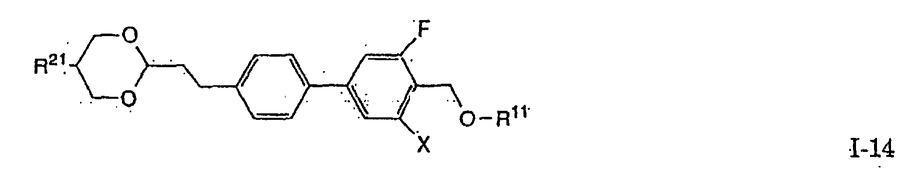 worin
- R¹¹: C₁-C₃-Alkyl;
- X: H oder F; und
- R²¹: C₂-C₆-Alkyl oder C₂-C₆-Alkenyl bedeuten.

Die Verbindungen der allgemeinen Formel I können in an sich bekannter Weise, wie in Schema 1 skizziert, hergestellt werden. Beispielsweise können Nitrile der allgemeinen Formel **1** durch saure Hydrolyse in die entsprechenden Säuren **3** umgewandelt werden. Auschliessende Reduktion der Säuren **3** zu den Alkoholen **4a** - **4g** und Veretherung von **4a** mit einem Alkylbromid oder -iodid führt direkt zu den Verbindungen der Formel I, während die Veretherung der Alkohole **4b** - **4g** zu den Zwischenprodukten der Formeln **5b** - **5g** fuhrt. Die Säuren der allgemeinen Formel **3** können ferner auch aus 3-Fluor- bzw. 3;5-Difluorphenylderivaten der Formel **2** durch Deprotonierung in 4-Stellung und anschliessende Carboxylierung mit CO₂ erhalten werden.

Durch saure Hydrolyse der Acetale oder Enolether der Formeln **4b** - **4g** werden zunächst die entsprechenden Ketone oder Aldehyde freigesetzt; diejenigen Verbindungen der Formel I, bei denen R² Alkyl bedeutet, lassen sich dann durch Wittig-Olefinierung, anschliessende Hydrierung und Veretherung herstellen. Für die Herstellung der Verbindungen der Formel I, bei denen R² Alkenyl bedeutet, wird zunächst in die Benzylether **5b** - **5g** umgewandelt, bevor - wie bei den Verbindungen der Formel **4b** - **4g** - die saure Hydrolyse durchgeführt wird. Die dadurch entstandenen Aldehyde oder Ketone lassen sich anschliessend, je nach gewünschter Lage der Doppelbindung gegebenenfalls durch Wittig-Reaktion mit Methoxymethyltriphenyl-phosphonium-Salzen und nachfolgender Hydrolyse der gebildeten Enolether ein- oder mehrfach homologisieren. Durch Reaktion mit Alkyltriphenylphosphonium Salzen können die Aldehyde in die gewünschten Olefine der Formel I übergeführt werden. Die Verbindungen der Formel I, bei denen der Ring B 1,3-Dioxan-2,5-diyl darstellt, können auf übliche Weise durch Acetalbildung der aus **5c**, **5d** oder **5f** gebildeten Aldehyde mit einem 2-Alkyl-1,3-propandiol oder einem analogen Alkenyl-1,3-propandiol hergestellt werden. Die ausgehend von **4b** - **4g** oder **5b** - **5g** erwähnten Reaktionen sind bekannt und in der Literatur der Flüssigkristallchemie beschrieben worden, beispielsweise in EP-A-0 122 389 oder in Molecular Crystals Liquid Crystals *131*, Seite 109ff oder Seite 327ff (1985).

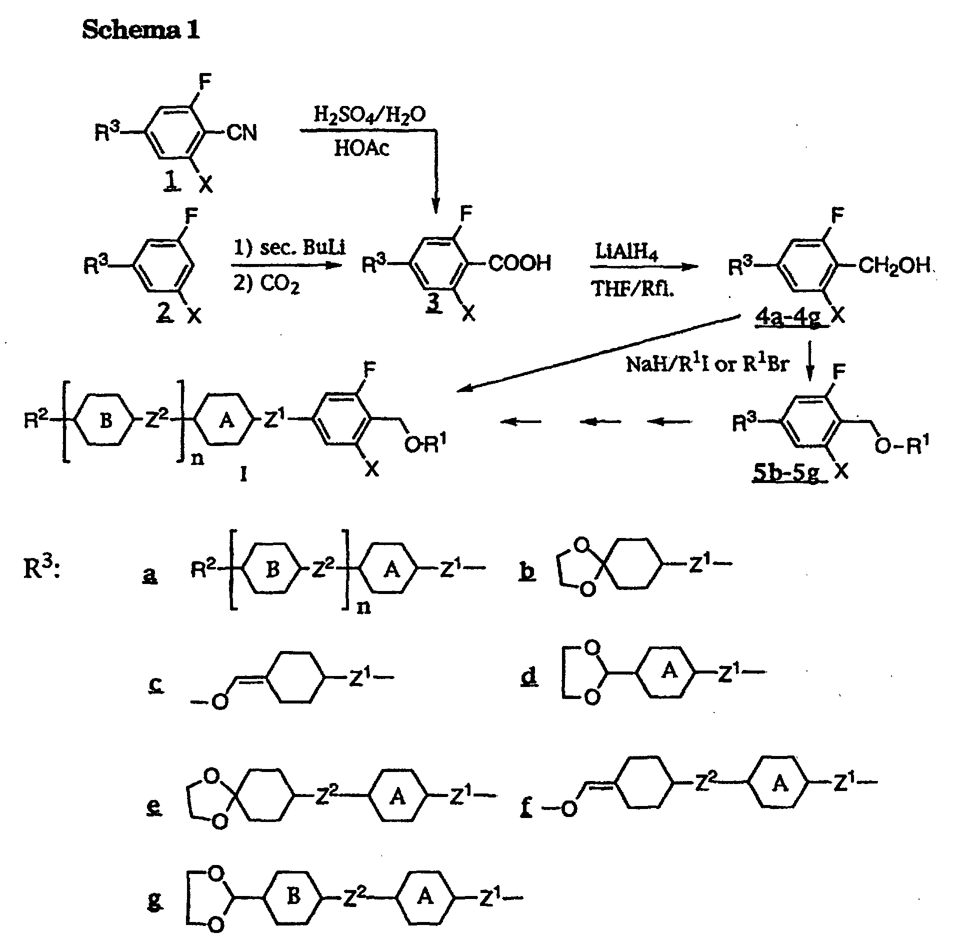

Die Herstellung der Verbindungen der allgemeinen Formel I, bei denen der Ring A einen aromatischen Ring darstellt, kann beispielsweise durch eine durch Palladium katalysierte Kupplung eines Boronsäurederivates mit einem aromatischen Bromid, Iodid oder auch Perfluoralkylsulfonat (z.B. Trifluormethylsulfonat) erfolgen, wie dies in Schema 2 dargestellt ist. Solche Kupplungen sind zum Beispiel in Tetrahedron Letters 35,3277 (1994) beschrieben worden.

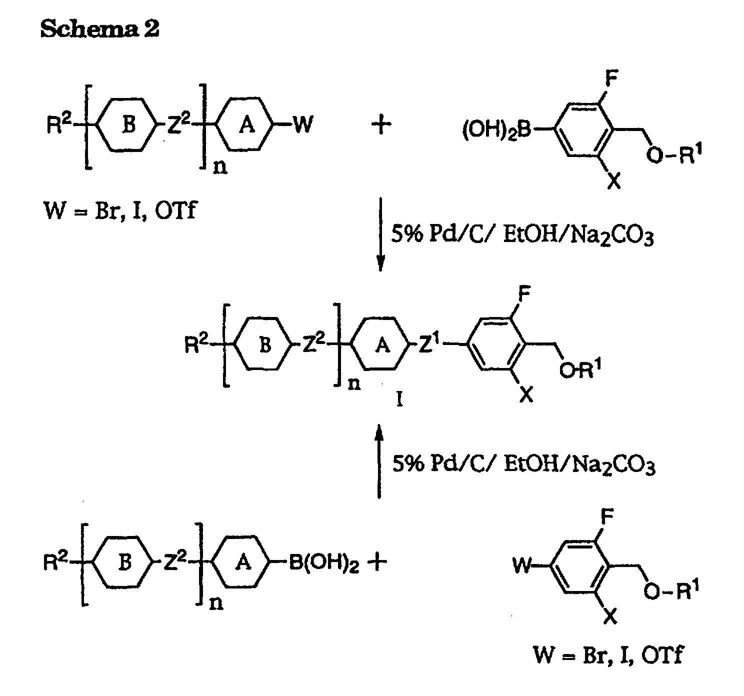

Die zur Synthese der Verbindungen der Formel I benötigten Ausgangsmaterialien sind bekannt oder Analoge von bekannten Verbindungen. So sind beispielsweise Nitrile der Formel **1** als Flüssigkristalle beschrieben worden. 3-Fluorphenyl- oder 3,5-Difluorphenyl-Derivate der Formel **2** sind als solche, als Isomere oder auch analoge Phenylfluoride ebenfalls beschrieben worden, beispielsweise in EP-A-0 315 014 oder EP-A-0 543 244.

Die Verbindungen der Formel I können in Form von Gemischen untereinander und/oder mit anderen Flüssigkristallkomponenten verwendet werden. Die Erfindung betrifft daher ebenfalls flüssigkristalline Gemische mit mindestens 2 Komponenten, welche dadurch gekennzeichnet sind, dass mindestens eine Komponente eine Verbindung der Formel I ist. Eine zweite und gegebenenfalls weitere Komponenten können weitere Verbindungen der allgemeinen Formel I oder andere geeignete Flüssigkristallkomponenten sein. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, oder aus Landolt-Börnstein, Liquid Crystals, Bände IV 7a-d, viele davon sind zudem im Handel erhältlich.

Aufgrund der guten Löslichkeit der erfindungsgemässen Verbindungen der Formel I in anderen Flüssigkristallmaterialien und aufgrund ihrer guten Mischbarkeit untereinander kann der Anteil an Verbindungen der Formel I in den erfindungsgemässen Gemischen relativ hoch sein und beispielsweise 1-70 Gew.-% betragen. Im allgemeinen ist ein Anteil von etwa 3-40 Gew.-%, insbesondere von etwa 5-20 Gew.-%, an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 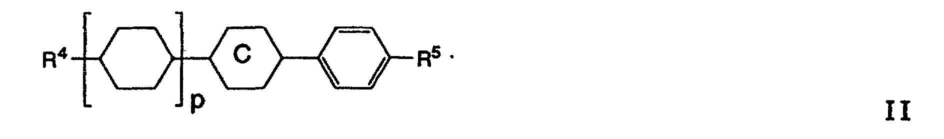 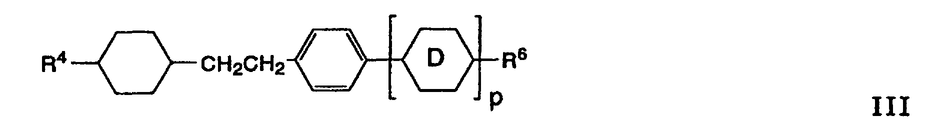 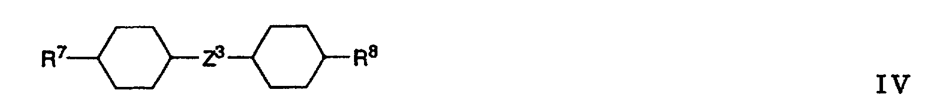 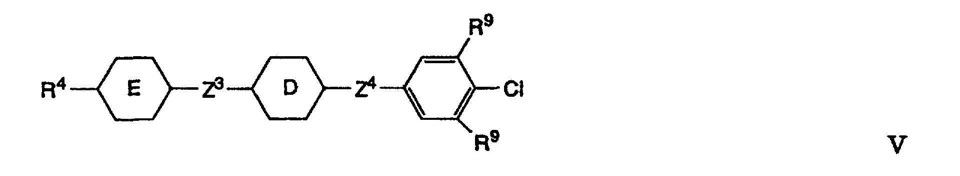 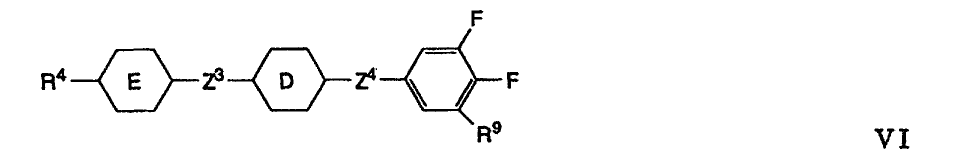 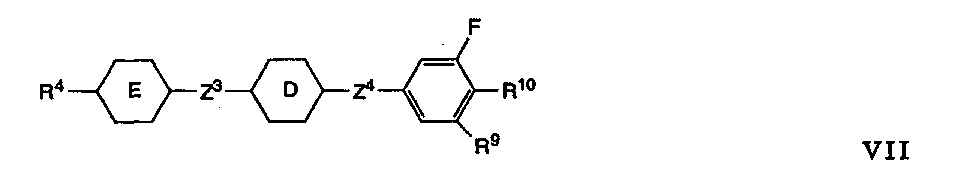 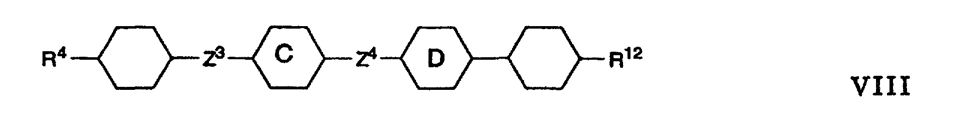 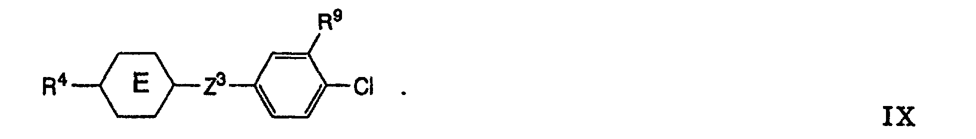 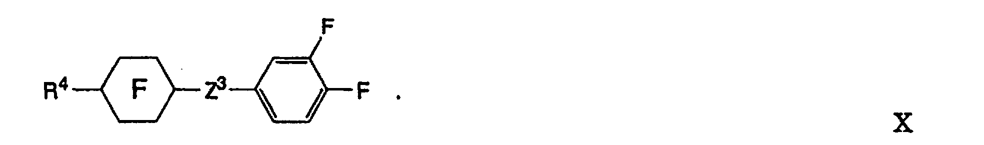 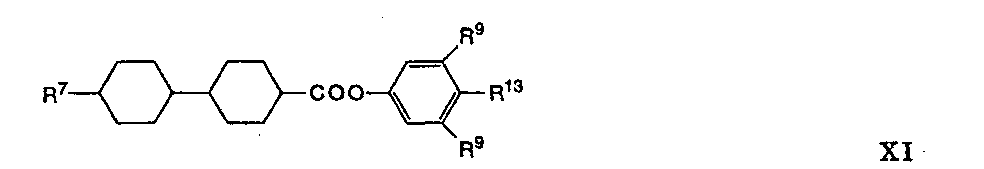 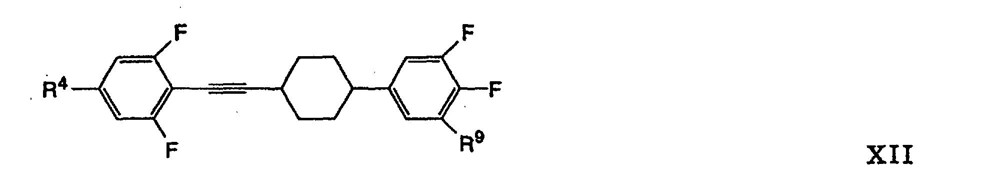 worin
- R⁴ und R¹²: Alkyl, Alkoxyalkyl, 3E-Alkenyl, 4-Alkenyl oder an einem gesättigten Ring auch 1E-Alkenyl;
- p: 0 oder 1;
- C: 1,4-Phenylen, Pyridin-2,5-diyl, Pyrimidin-2,5-diyl, trans-1,4-Cyclohexylen oder trans-1,3-Dioxan-2,5-diyl;
- R⁵: Cyano, Isothiocyanato, Fluor, Alkyl, 3E-Alkenyl, 4-Alkenyl, Alkoxy, 2E-Alkenyloxy, 3-Alkenyloxy oder 1-Alkinyl;
- D: 1,4-Phenylen oder trans-1,4-Cyclohexylen;
- R⁶: Alkyl, 3E-Alkenyl, 4-Alkenyl, oder an trans-1,4-Cyclohexylen auch 1E-Alkenyl, oder an 1,4-Phenylen auch Cyano, Isothiocyanato, Alkoxy, 2E-Alkenyloxy oder 3-Alkenyloxy;
- R⁷: Alkyl, 1E-Alkenyl, 3E-Alkenyl oder 4-Alkenyl;
- R⁸: Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl;
- Z³ und Z⁴: eine Einfachbindung oder -CH₂CH₂-, wobei zwei aromatische Ringe immer durch eine Einfachbindung verknüpft sind;
- E: trans-1,4-Cyclohexylen oder trans-1,3-Dioxan-2,5-diyl;
- R⁹: Wasserstoff oder Fluor;
- R¹⁰: Difluormethoxy oder Trifluormethoxy;
- R¹³: Cyano, Fluor, Chlor, Difluormethoxy oder Trifluormethoxy; und
- F: Pyrimidin-2,5-diyl, trans-1,4-Cyclohexylen oder trans-1,3-Dioxan-2,5-diyl bedeuten.

Die im Zusammenhang mit den Verbindungen der Formeln II bis XVI verwendeten Ausdrücke Alkyl, Alkyloxy, 1E-Alkenyl, 3E-Alkenyl und Alkenyl mit endständiger Doppelbindung sind weiter oben definiert; "4-Alkenyl" bedeutet vorzugsweise geradkettige Alkenylreste mit höchstens 12 Kohlenstoffatomen, in denen sich die Doppelbindung in 4-Stellung befindet, wie beispielsweise 4-Pentenyl, 4-Hexenyl oder 4-Heptenyl.
"Aromatische Ringe" bedeutet Ringe, wie beispielsweise 1,4-Phenylen, Pyridin-2,5-diyl oder Pyrimidin-2,5-diyl.
"Gesättigte Ringe" bedeutet trans-1,4-Cyclohexylen oder trans-1,3-Dioxan-2,5-diyl.
"Alkyloxyalkyl" bedeutet vorzugsweise geradkettige Reste mit höchstens 12 Kohlenstoffatomen, wie beispielsweise Methoxymethyl, Ethoxymethyl, Propyloxymethyl, Butyloxymethyl und dergleichen.
"2E- bzw. 3-Alkenyloxy" bedeutet vorzugsweise geradkettige Alkenyloxyreste mit höchstens 12 Kohlenstoffatomen, in denen sich die Doppelbindung in 2-bzw 3-Stellung befindet und E bzw. Z die bevorzugte Konfiguration angibt, wie beispielsweise Allyloxy, 2E-Butenyloxy, 2E-Pentenyloxy, 2E-Hexenyloxy, 2E-Heptenyloxy, 3-Butenyloxy, 3-Pentenyloxy, 3-Hexenyloxy, 3-Heptenyloxy, 4-Pentenyloxy, 5-Hexenyloxy, 6-Heptenyloxy und dergleichen.
"1-Alkinyl" bedeutet vorzugsweise geradkettige Alkinylreste mit höchstens 12 Kohlenstoffatomen, in denen die Dreifachbindung sich in 1-Stellung befindet, wie beispielsweise Ethinyl, 1-Propinyl, 1-Butinyl, 1-Pentinyl und dergleichen.

Die erfindungsgemässen Mischungen können ferner optisch aktive Verbindungen (z.B. optisch aktive 4'-Alkyl- oder 4'-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe der Helix, Farbe, Extinktion und dergleichen bestimmt. Im allgemeinen beträgt der Anteil optisch aktiver Verbindungen und dichroitischer Farbstoffe höchstens je etwa 10 Gew.% im Gesamtgemisch.

Die Herstellung der flüssigkristallinen Gemische und der elektro-optischen Vorrichtung kann in an sich bekannter Weise erfolgen.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische Phase, N eine nematische und I die isotrope Phase. V₁₀ bezeichnet die Spannung für 10% Transmission (Blickrichtung senkrecht zur Plattenoberfläche). tₒₙ und t_{off} bezeichnen die Einschaltzeit bzw. die Ausschaltzeit und Δn die optische Anisotropie.

### Beispiel 1

a) Zu 14,4 g (0,623 Mol) Magnesiumspänen und einer Spatelspitze Iod-Kristallen wurde eine Lösung von 109g 1-Brom-3-fluorbenzol in 270 ml trockenem Tetrahydrofuran langsam zugetropft, so dass das Reaktionsgemisch bei Rückflusstemperatur gehalten wurde. Das Reaktionsgemisch wurde bis zum vollständigen Verbrauch des Magnesiums bei 73-75°C gelassen, dann auf 5°C abgekühlt und innert 20 Min. mit einer Lösung von 117,2 g 4-(1,4-Dioxaspiro[4,5]dec-8-yl)cyclohexanon in 460 ml wasserfreiem Tetrahydrofuran bei 5-15°C versetzt. Danach wurde die Reaktionslösung auf Raumtemperatur erwärmt und 90 Min. gerührt. Anschliessend wurden 800 ml 10proz. Ammoniumchlorid Lösung so zugetropft, dass die Temperatur 35°C nicht überstieg. Das Reaktionsgemisch wurde hierauf mit Ether extrahiert, die organische Phase über Natriumsulfat getrocknet, filtriert und das Filtrat eingedampft. Dies ergab 174,3 g rohes 4-(1,4-Dioxaspiro[4,5]dec-8-yl)-1-(3-fluorophenyl)cyclohexanol als bräunliche Kristalle, welche ohne weitere Reinigung in der nächsten Reaktion eingesetzt wurden. (cis/trans: 47.5:49.5).
b) Ein Gemisch von 174,3 g 4-(1,4-Dioxaspiro[4,5]dec-8-yl)-1-(3-fluorophenyl)cyclohexanol, 1000 ml 1,2-Dichlorethan, 25 ml Ethylenglycol und 25 g Amberlyst 15 wurde während 22 Std. unter Rückfluss gekocht, wobei das Kondensat über einen mit 175 g Alox I, neutral, gefüllten Tropftrichter getrocknet wurde. Dann wurde das Reaktionsgemisch abgekühlt, filtriert, mit Wasser gewaschen und die organische Phase über Natriumsulfat getrocknet. Nachfolgende Chromatographie an 1000 g Kieselgel mit Hexan/Essigester (9:1) ergab 124,2 g 4-(1,4-Dioxaspiro[4,5]dec-8-yl)-1-(3-fluorophenyl)cyclohexen als farblose Kristalle. Smp. einer aus Methylenchlorid/Hexan umkristallisierten Probe: 133,7-135,3 °C.
c) Die Lösung von 4-(1,4-Dioxaspiro[4,5]dec-8-yl)-1-(3-fluorophenyl)cyclohexen in 2 1 Toluol wurde mit 10% Pd/C bei Raumtemperatur und unter Normaldruck bis zur Aufnahme von 12,4 l Wasserstoff hydriert. Das Reaktionsgemisch wurde hierauf filtriert und das Filtrat eingedampft. Dies ergab 125,3 g 4-(1,4-Dioxaspiro[4,5]dec-8-yl)-1-(3-fluorophenyl)cyclohexan als gelbliches Oel (cis/trans 50/47).
d) Zur einer Lösung von 125,3 g 4-(1,4-Dioxaspiro[4,5]dec-8-yl)-1-(3-fluorophenyl)cyclohexan (cis/trans 50/47) in 630 ml Dimethylsulfoxyd wurden 23,7g Kalium-t.-Butylat zugesetzt, die Mischung während 2 Std. bei 100°C gerührt, abgekühlt und auf 2500 ml 5 proz. Kochsalzlösung gegossen. Das Reaktionsgemisch wurde mit Methylenchlorid extrahiert, die organsiche Phase über Natriumsulfat getrocknet, filtriert und das Filtrat eingedampft. Kristallisation des Rückstandes aus Isopropanol ergab 121,9 g trans-4-(1,4-Dioxaspiro[4,5]dec-8-yl)-1-(3-fluorophenyl)cyclohexan als farblose Kristalle (trans Gehalt 99.8%).
e) Unter Argon wurde eine Lösung von 6,3 g trans-4-(1,4-Dioxaspiro[4,5]dec-8-yl)-1-(3-fluorophenyl)cyclohexan und 2,317 g TMEDA in 50 ml trockenem Tetrahydrofuran bei -75°C innert 15 Min. mit 25 ml (32,5 mMol) einer sec. Butyllithium Lösung versetzt. Dann wurde 2,5 Std. bei derselben Temperatur nachreagieren gelassen. Während dieser Zeit wurde zu 300 ml Ether soviel festes Kohlendioxyd eingetragen, dass die Gewichtszunahme der Lösung 40 g betrug. In diese Etherlösung wurde die Tetrahydrofuran-Lösung mittels Katheter eingedrückt, wobei die Temperatur auf -52°C stieg. Das Reaktionsgemisch wurde auf 0°C erwärmt, und vorsichtig mit 100 ml eiskalter 1N Salzsäure und anschliessend 200 ml Eiswasser versetzt, mit Methylenchlorid extrahiert, die organische Phase über Natriumsulfat getrocknet, filtriert und das Filtrat eingedampft. Der Rückstand wurde aus Aceton/Hexan kristallisiert. Dies ergab 3,4 g 4-[trans-4-(1,4-Dioxaspiro[4,5]dec-8-yl)cyclohexyl]-2-fluorbenzoesäure als gelbliche Kristalle (Smp. 227.8°C zers.).
f) Zu einer Suspension von 3 g Lithiumaluminiumhydrid in 100 ml trockenem Ether wurde 5,7 g 4-[trans-4-(1,4-Dioxaspiro[4,5]dec-8-yl)cyclohexyl]-2-fluorbenzoesäure zugesetzt und nach 23 Std. Rühren unter Rückfluss auf 0°C gekühlt, vorsichtig mit 25 ml Wasser und dann 100 ml 3N Schwefelsäure versetzt. Das Reaktionsgemisch wurde hierauf mit Ether extrahiert, die organische Phase über Natriumsulfat getrocknet, filtriert und das Filtrat eingedampft. Chromatographie des Rückstandes an Kieselgel mit Methylenchlorid/Aceton (95:5) ergab 3,1 g 4-[trans-4-(1,4-Dioxaspiro[4,5]dec-8-yl)cyclohexyl]-2-fluorbenzylalkohol als farblose Kristalle.
g) Eine Lösung von 3,1 g 4-[trans-4-(1,4-Dioxaspiro[4,5]dec-8-yl)cyclohexyl]-2-fluorbenzylalkohol in 100 ml Toluol und 40 ml Ameisensäure wurde 4 Std. bei Raumtemperatur gerührt, auf Wasser gegossen, mit Toluol extrahiert, die organischen Phasen vereinigt, mit 10 proz. Natriumbicarbonat gewaschen, über Natriumsulfat getrocknet, filtriert und das Filtrat eingedampft. Dies ergab 3,06 g rohes 4-[trans-4-(4-Oxocyclohexyl)cyclohexyl]-2-fluorbenzylformiat von 99% Reinheit (GC).
h) Zu einer Suspension von 6,6 g Methoxymethyltriphenylphosphoniumchlorid in 40 ml t.-Butylmethylether wurden bei -15°C 2,7 g Kalium-t.-butylat zugesetzt und während einer Std. gerührt. Dann wurde bei 0°C mit 20 ml t.-Butylmethyläther verdünnt und hierauf eine Lösung von 3,88 g 4-[trans-4-(4-Oxocyclohexyl)cyclohexyl]-2-fluorbenzylformiat in 40 ml Tetrahydrofuran während 50 Min. zugetropft. Nach weiteren 2 Std. bei 0°C wurde die Reaktionslösung auf Wasser gegossen und das Gemisch mit Methylenchlorid extrahiert. Die organische Phase wurde über Natriumsulfat getrocknet, filtriert und das Filtrat eingeengt. Der Rückstand wurde an Kieselgel mit Methylenchlorid/Aceton (98:2) chromatographiert. Dies ergab 0,69 g 4-[trans-4-(4-Methoxymethylidencyclohexyl)cyclohexyl]-2-fluorbenzylalkohol neben 1,82 g 4-[trans-4-(4-Oxocyclohexyl)cyclohexyl]-3-fluorbenzylalkohol, welcher erneut eingesetzt werden konnte.
i) 0,4 g Natriumhydrid-Dispersion (in Oel, ca 50 proz.) wurde mit Hexan gewaschen, mit einer Lösung von 1,11 g 4-[trans-4-(4-Methoxymethylidencyclohexyl)cyclohexyl]-2-fluorbenzylalkohol versetzt, und anschliessend wurden 0,44 ml Methyljodid zugesetzt. Das Gemisch wurde 1 Std. bei Rückflusstemperatur gehalten, gekühlt, vorsichtig mit Wasser versetzt und das Reaktionsgemisch mit Ether extrahiert. Die organische Phase wurde über Natriumsulfat getrocknet, filtriert und das Filtrat eingedampft. Dies ergab 1,15 g 4-[trans-4-(4-Methoxymethylidencyclohexyl)-cyclohexyl]-2-fluorbenzylmethylether als gelbliches Oel, (Reinheit gemäss GC 100%).
j) Eine Lösung von 1,15 g 4-[trans-4-(4-Methoxymethylidencyclohexyl)-cyclohexyl]-2-fluorbenzylmethylether in 10 ml Tetrahydrofuran wurde mit 2,5 ml einer 3N Salzsäure versetzt und 30 Min. bei Rückflusstemperatur gerührt. Die Reaktionslösung wurde abgekühlt, zwischen Methylenchlord und 10 proz. Natriumbicarbonatlösung verteilt, die organischen Phasen über Natriumsulfat getrocknet, filtriert und das Filtrat eingedampft. Dies ergab 1,11 g 4-[trans-4-(4-Formylcyclohexyl)cyclohexyl]-2-fluorbenzylmethylether (cis/trans = 4:1).
k) Eine Lösung von 1,11 g 4-[trans-4-(4-Formylcyclohexyl)cyclohexyl]-2-fluorbenzylmethylether (cis/trans = 4:1) in 1,5 ml Methylenchlorid wurde nacheinander mit einem Tropfen Triethylamin, 12 ml Methanol und 0,45 ml 20 proz. Natronlauge versetzt und während 30 Min. bei Raumtemperatur gerührt. Dann wurde das Reaktionsgemisch auf 50 ml Wasser gegossen und mit Methylenchlorid extrahiert. Die organische Phase wurde über Natriumcarbonat getrocknet, filtriert und das Flitrat eingedampft. Kristallisation aus Hexan ergab 0,53 reinen 4-[trans-4-(trans-4-Formylcyclohexyl)cyclohexyl]-2-fluorbenzylmethylether, Smp. 52-54°C.
l) Eine Suspension von 0,41 g Methyltriphenylphosphoniumbromid in 10 ml t.-Butylmethylether wurde bei 0°C mit 0,135 g Kalium-t.-butylat versetzt. Nach 5 Min. wurde eine Lösung von 0,25 g 4-[trans-4-(trans-4-Formylcyclohexyl)cyclohexyl]-2-fluorbenzylmethylether in 5 ml Kalium-t.butylat zugetropft, das Reaktionsgemisch weitere 30 Min. bei 0°C gerührt, auf 50 ml Wasser gegossen, mit Ether extrahiert, die organischen Phasen vereinigt, über Natriumsulfat getrocknet, filtriert und das Filtrat eingedampft. Der Rückstand wurde an Kieselgel mit Methylenchlorid chromatographiert und aus Methylenchlorid/Isopropanol und Hexan/Isopropanol kristallisiert. Dies ergab 0,165 g 4-[trans-4-(trans-4-Vinylcyclohexyl)cyclohexyl]-2-fluorbenzylmethylether mit folgenden Eigenschaften: Smp. (K/N): 30,9 °C, Klp. (N/I): 114,2 °C.

Auf analoge Weise können die folgenden Verbindungen hergestellt werden:
4-[trans-4-(trans-4-(E)-Propenylcyclohexyl)cyclohexyl]-2-fluorbenzylmethylether
4-[trans-4-(trans-4-(E)-(1-Butenyl)cyclohexyl)cyclohexyl]-2-fluorbenzylmethylether
4-[trans-4-(trans-4-(E)-(1-Pentenyl)cyclohexyl)cyclohexyl]-2-fluorbenzylmethylether
4-{trans-4-[2-(trans-4-Vinylcyclohexyl)ethyl]cyclohexyl}-2-fluorbenzylmethylether
4-{trans-4-[2-(trans-4-(E)-Propenylcyclohexyl)ethyl]cyclohexyl}-2-fluorbenzylmethylether
4-{trans-4-[2-(trans-4-(E)-(1-Pentenyl)cyclohexyl)ethyl]cyclohexyl}-2-fluorbenzylmethylether
4-[trans-4-(trans-4-Vinylcyclohexyl)cyclohexyl]-2,6-difluorbenzylmethylether, Smp. (K/N): 67 °C, Klp. (N/I): 67,5 °C.
4-[trans-4-(trans-4-(E)-Propenylcyclohexyl)cyclohexyl]-2,6-difluorbenzylmethylether
4-[trans-4-(trans-4-(E)-(1-Butenyl)cyclohexyl)cyclohexyl]-2,6-difluorbenzylmethylether
4-[trans-4-(trans-4-(E)-(1-Pentenyl)cyclohexyl)cyclohexyl]-2,6-difluorbenzylmethylether
4-{trans-4-[2-(trans-4-Vinylcyclohexyl)ethyl]cyclohexyl}-2,6-difluorbenzylmethylether
4-{trans-4-[2-(trans-4-(E)-Propenylcyclohexyl)ethyl]cyclohexyl}-2,6-difluorbenzylmethylether
4-{trans-4-[2-(trans-4-(E)-(1-Pentenyl)cyclohexyl)ethyl]cyclohexyl}-2,6-difluorbenzylmethylether
4-[trans-4-(trans-4-Vinylcyclohexyl)cyclohexyl]-2-fluorbenzylethylether
4-[trans-4-(trans-4-(E)-(1-Butenyl)cyclohexyl)cyclohexyl]-2-fluorbenzylethylether
4-[trans-4-(trans-4-(E)-(1-Pentenyl)cyclohexyl)cyclohexyl]-2-fluorbenzylethylether
4-[trans-4-(trans-4-Vinylcyclohexyl)cyclohexyl]-2,6-difluorbenzylethylether
4-[trans-4-(trans-4-(E)-Propenylcyclohexyl)cyclohexyl]-2,6-difluorbenzylethylether
4-[trans-4-(trans-4-(E)-(1-Butenyl)cyclohexyl)cyclohexyl]-2,6-difluorbenzylethylether
4-[trans-4-(trans-4-(E)-(1-Pentenyl)cyclohexyl)cyclohexyl]-2,6-difluorbenzylethylether.

### Beispiel 2

Eine Lösung von 2 g 4-(trans-4-Formylcyclohexyl)-3-fluorbenzylmethylether, hergestellt analog zu Beispiel 1 (a)-k)) und 1,05 g 2-Propyl-1,3-propandiol in 30 ml Benzol wird mit 35 mg p-Toluolsulfonsäure-monohydrat 1 Std. zu leichtem Sieden erhitzt. Nach Neutralisation der Lösung mit einigen Tropfen Triethylamin wird mit Wasser gewaschen, über Natriumsulfat getrocknet, filtriert und das Filtrat eingedampft. Chromatographie des Rückstandes an Kieselgel mit Methylenchlorid und anschliessende Kristallisation aus Hexan ergibt 4-[trans-4-(trans-5-Propyl-1,3-dioxan-2-yl)cyclohexyl]-2-fluorbenzylmethylether.
Auf analoge Weise können hergestellt werden:
4-[trans-4-(trans-5-Propyl-1,3-dioxan-2-yl)cyclohexyl]-2-fluorbenzylmethylether
4-[trans-4-(trans-5-Butyl-1,3-dioxan-2-yl)cyclohexyl]-2-fluorbenzylmethylether
4-[trans-4-(trans-5-Pentyl-1,3-dioxan-2-yl)cyclohexyl]-2-fluorbenzylmethylether
4-[trans-4-(trans-5-Vinyl-1,3-dioxan-2-yl)cyclohexyl]-2-fluorbenzylmethylether
4-[trans-4-(trans-5-(E-)Propenyl-1,3-dioxan-2-yl)cyclohexyl]-2-fluorbenzylmethylether
4-[trans-4-(trans-5-(3-Butenyl)-1,3-dioxan-2-yl)cyclohexyl]-2-fluorbenzylmethylether
4-[trans-4-(trans-5-Propyl-1,3-dioxan-2-yl)cyclohexyl]-2,6-difluorbenzylmethylether
4-[trans-4-(trans-5-Butyl-1,3-dioxan-2-yl)cyclohexyl]-2,6-difluorbenzylmethylether
4-[trans-4-(trans-5-Pentyl-1,3-dioxan-2-yl)cyclohexyl]-2,6-difluorbenzylmethylether
4-[trans-4-(trans-5-Vinyl-1,3-dioxan-2-yl)cyclohexyl]-2,6-difluorbenzylmethylether
4-[trans-4-(trans-5-(E-)Propenyl-1,3-dioxan-2-yl)cyclohexyl]-2,6-difluorbenzylmethylether
4-[trans-4-(trans-5-(3-Butenyl)-1,3-dioxan-2-yl)cyclohexyl]-2,6-difluorbenzylmethylether
4-{2-[trans-4-(trans-5-Propyl-1,3-dioxan-2-yl)cyclohexyl]ethyl}-2-fluorbenzylmethylether
4-{2-[trans-4-(trans-5-Pentyl-1,3-dioxan-2-yl)cyclohexyl]ethyl}-2-fluorbenzylmethylether
4-{2-[trans-4-(trans-5-Vinyl-1,3-dioxan-2-yl)cyclohexyl]ethyl}-2-fluorbenzylmethylether
4-{2-[trans-4-(trans-5-(3-Butenyl)-1,3-dioxan-2-yl)cyclohexyl]ethyl}-2-fluorbenzylmethylether
4-{2-[trans-4-(trans-5-Propyl-1,3-dioxan-2-yl)cyclohexyl]ethyl}-2,6-difluorbenzylmethylether
4-(2-[trans-4-(trans-5-Pentyl-1,3-dioxan-2-yl)cyclohexyl]ethyl}-2,6-difluorbenzylmethylether
4-{2-[trans-4-(trans-5-Vinyl-1,3-dioxan-2-yl)cyclohexyl]ethyl}-2,6-difluorbenzylmethylether
4-{2-[trans-4-(trans-5-(3-Butenyl)-1,3-dioxan-2-yl)cyclohexyl]ethyl}-2,6-difluorbenzylmethylether
4-[trans-4-(trans-5-Propyl-1,3-dioxan-2-yl)cyclohexyl]-2-fluorbenzylethylether
4-[trans-4-(trans-5-Butyl-1,3-dioxan-2-yl)cyclohexyl]-2-fluorbenzylethylether
4-[trans-4-(trans-5-Pentyl-1,3-dioxan-2-yl)cyclohexyl] -2-fluorbenzylethylether
4-[trans-4-(trans-5-Propyl-1,3-dioxan-2-yl)cyclohexyl]-2-fluorbenzylallylether
4-[trans-4-(trans-5-Butyl-1,3-dioxan-2-yl)cyclohexyl]-2-fluorbenzylallylether
4-[trans-4-(trans-5-Pentyl-1,3-dioxan-2-yl)cyclohexyl]-2-fluorbenzylallylether.

### Beispiel 3

a) Eine Suspension von 20 g 4-[trans-4-(trans-4-Propylcyclohexyl)cyclohexyl]-2-fluorbenzonitril in 400 ml Eisessig wird mit 200 ml 50 proz. Schwefelsäure versetzt und während 20 Std. auf Rückflusstemperatur erhitzt. Dann wird abgekühlt, mit festem Natriumhydroxyd of pH 4 gestellt und das Reaktionsgemisch mit Methylenchlorid extrahiert. Die organischen Phasen werden vereinigt, mit Wasser gewaschen, über Natriumsulfat getrocknet, filtriert und das Filtrat eingedampft. Dies ergibt 4-[trans-4-(trans-4-Propylcyclohexyl)cyclohexyl]-2-fluorbenzoesäure.
b) Zu einer Suspension von 4,5g Lithiumaluminiumhydrid in 150ml trockenem Ether wird 7,8 g 4-[trans-4-(trans-4-Propyl)cyclohexyl]-2-fluorbenzoesäure zugesetzt und unter Rückfluss 23 Std. gerührt. Die Suspension wird auf 0°C gekühlt, vorsichtig mit 40 ml Wasser und dann mit 150 ml 3N Schwefelsäure versetzt, mit Ether extrahiert und die organische Phase über Natriumsulfat getrocknet, filtriert und das Filtrat eingedampft. Chromatographie des Rückstandes an Kieselgel mit Methylenchlorid/Aceton (95:5) ergibt 4-[trans-4-(trans-4-Propylcyclohexyl)cyclohexyl]-2-fluorbenzylalkohol.
c) 1,2 g Natriumhydrid-Dispersion (in Oel, ca 50 proz.) wird mit Hexan gewaschen und dann mit einer Lösung von 3,33 g 4-[trans-4-(trans-4-Propylcyclohexyl)cyclohexyl]-2-fluorbenzylalkohol versetzt. Hierauf werden 1,32 ml Methyljodid zugesetzt und das Gemisch 1 Std. bei Rückflusstemperatur gehalten, abgekühlt, vorsichtig mit Wasser versetzt und mit Ether extrahiert. Die organische Phase wird hierauf über Natriumsulfat getrocknet, filtriert und das Filtrat abgedampft. Der Rückstand wird an Kieselgel mit Methylenchlorid chromatographiert und aus Methylenchlorid/Isopropanol und Hexan/Isopropanol kristallisiert. Dies ergibt 4-[trans-4-(trans-4-Propylcyclohexyl)cyclohexyl]-2-fluorbenzylmethylether.
Auf analoge Weise können die folgenden Verbindungen hergestellt werden:
4-[trans-4-(trans-4-Ethylcyclohexyl)cyclohexyl]-2-fluorbenzylmethylether
4-[trans-4-(trans-4-Butylcyclohexyl)cyclohexyl] -2-fluorbenzylmethylether
4- [trans-4-(trans-4-(3-Butenyl)cyclohexyl)cyclohexyl]-2-fluorbenzylmethylether, Smp. (K/N): 83.2°C, Klp. (N/I): 139.9°C
4-[trans-4-(trans-4-Pentylcyclohexyl)cyclohexyl]-2-fluorbenzylmethylether
4-[trans-4-(trans-4-(4-Pentenyl)cyclohexyl)cyclohexyl]-2-fluorbenzylmethylether
4-[trans-4-(trans-4-Ethylcyclohexyl)cyclohexyl]-2,6-difluorbenzylmethylether
4-[trans-4-(trans-4-Propylcyclohexyl)cyclohexyl]-2,6-difluorbenzylmethylether
4-[trans-4-(trans-4-Butylcyclohexyl)cyclohexyl]-2,6-difluorbenzylmethylether
4-[trans-4-(trans-4-(3-.Butenyl)cyclohexyl)cyclohexyl]-2,6-difluorbenzylmethylether, Smp. (K/N): 54.9 °C, Klp. (N/I): 105 °C
4-[trans-4-(trans-4-Pentylcyclohexyl)cyclohexyl]-2,6-difluorbenzylmethylether
4-[trans-4-(terans-4-(4-Pentenyl)cyclohexyl)cyclohexyl]-2,6-difluorbenzylmethylether
4-{trans-4-[2-(trans-4-Ethylcyclohexyl)ethyl]cyclohexyl}-2-fluorbenzylmethylether
4-{trans-4-[2-(trans-4-Propylcyclohexyl)ethyl]cyclohexyl}-2-fluorbenzylmethylether
4-{trans-4-[2-(trans-4-(3-Butenyl)cyclohexyl)ethyl]cyclohexyl}-2-fluorbenzylmethylether
4-{trans-4-[2-(trans-4-(Pentylcyclohexyl)ethyl]cyclohexyl}-2-fluorbenzylmethylether
4-{trans-4-[2-(trans-4-Ethylcyclohexyl)ethyl] cyclohexyl}-2,6-difluorbenzylmethylether
4-{trans-4-[2-(trans-4-Propylcyclohexyl)ethyl]cyclohexyl}-2,6-difluorbenzylmethylether
4-{trans-4-[2-(trans-4-(3-Butenyl)cyclohexyl)ethyl]cyclohexyl}-2,6-difluorbenzylmethylether
4-{trans-4-[2-(trane-4-(Pentylcyclohexyl)ethyl]cyclohexyl}-2,6-difluorbenzylmethylether
4-{2-[trans-4-(trans-4-Ethylcyclohexyl)cyclohexyl]ethyl}-2-fluorbenzylmethylether
4-{2-[trans-4-(trans-4-Propylcyclohexyl)cyclohexyl]ethyl}-2-fluorbenzylmethylether
4-{2-[trans-4-(trans-4-Butylcyclohexyl)cyclohexyl]ethyl}-2-fluorbenzylmethylether
4-{2-[trans-4-(trans-4-Pentylcyclohexyl)cyclohexyl]ethyl}-2-fluorbenzylmethylether
4-{2-[trans-4-(trans-4-Vinylcyclohexyl)cyclohexyl]ethyl}-2-fluorbenzylmethylether
4-{2-[trans-4-(trans-4-(E)-Propenylcyclohexyl)cyclohexyl]ethyl}-2-fluorbenzylmethylether
4-{2-[trans-4-(trans-4-(3-Butenyl)cyclohexyl)cyclohexyl]ethyl}-2-fluorbenzylmethylether
4-{2-[trans-4-(trans-4-(4-Pentenyl)cyclohexyl)cyclohexyl]ethyl}-2-fluorbenzylmethylether
4-{2-[trans-4-(trans-4-Ethylcyclohexyl)cyclohexyl]ethyl}-2,6-difluorbenzylmethylether
4-{2-[trans-4-(trans-4-Propylcyclohexyl)cyclohexyl]ethyl}-2,6-difluorbenzylmethylether
4-{2-[trans-4-(trans-4-Butylcyclohexyl)cyclohexyl]ethyl}-2,6-difluorbenzylmethylether
4-{2-[trans-4-(trans-4-Pentylcyclohexyl)cyclohexyl]ethyl}-2,6-difluorbenzylmethylether
4-{2-[trans-4-(trans-4-Vinylcyclohexyl)cyclohexyl]ethyl}-2,6-difluorbenzylmethylether, Smp. (K/N): 56.6°C, Klp. (N/I): 77°C
4-{2-[trans-4-(trans-4-(E)-Propenylcyclohexyl)cyclohexyl]ethyl}-2,6-difluorbenzylmethylether
4-{2-[trans-4-(trans-4-(3-Butenyl)cyclohexyl)cyclohexyl]ethyl}-2,6-difluorbenzylmethylether, Smp. (C/N): 40.2°C, Klp. (N/I): 101.2°C
4-{2-[trans-4-(trans-4-(4-Pentenyl)cyclohexyl)cyclohexyl]ethyl}-2,6-difluorbenzylmethylether
4-[trans-4-(trans-4-Ethylcyclohexyl)cyclohexyl]-2-fluorbenzylethylether
4-[trans-4-(trans-4-Butylcyclohexyl)cyclohexyl]-2-fluorbenzylethylether
4-[trans-4-(trans-4-Pentylcyclohexyl)cyclohexyl]-2-fluorbenzylethylether
4-[trans-4-(trans-4-Ethylcyclohexyl)cyclohexyl]-2,6-difluorbenzylethylether
4-[trans-4-(trans-4-Propylcyclohexyl)cyclohexyl]-2,6-difluorbenzylethylether
4-[trans-4-(trans-4-Butylcyclohexyl)cyclohexyl]-2,6-difluorbenzylethylether
4-[trans-4-(trans-4-Pentylcyclohexyl)cyclohexyl]-2,6-difluorbenzylethylether
4-[trans-4-(trans-4-Ethylcyclohexyl)cyclohexyl]-2-fluorbenzylallylether
4-[trans-4-(trans-4-Butylcyclohexyl)cyclohexyl]-2-fluorbenzylallylether
4-[trans-4-(trans-4-Pentylcyclohexyl)cyclohexyl]-2-fluorbenzylallylether
4-[trans-4-(trans-4-Ethylcyclohexyl)cyclohexyl]-2,6-difluorbenzylallylether
4-[trans-4-(trans-4-Propylcyclohexyl)cyclohexyl]-2,6-difluorbenzylallylether
4-[trans-4-(trans-4-Butylcyclohexyl)cyclohexyl]-2,6-difluorbenzylallylether
4-[trans-4-(trans-4-Pentylcyclohexyl)cyclohexyl]-2,6-difluorbenzylallylether

### Beispiel 4

Ein Gemisch von 5,62 g 4-(trans-4-Propylcyclohexyl)brombenzol, 4,05 g 3-Fluor-4-methoxymethylphenylboronsäure, 430 mg 5 proz. Palladiumkohle (DEGUSSA E 101 N/D), 50 ml Benzol, 25 ml Ethanol und 50 ml 2M Natriumcarbonatlösung werden 3 Std. zum Sieden erhitzt, vom Katalysator abfiltriert und die wässrige Phase abgetrennt. Die organische Phase wird mit 1N Natriumhydroxid Lösung, gesättigter Natriumhydrogencarbonatlösung und mit Wasser gewaschen, über Natriumsulfat getrocknet, filtriert und das Filtrat eingedampft. Dies ergibt 4-[4-(trans-4-Propylcyclohexyl)phenyl]-2-fluorbenzylmethylether.
Auf analoge Weise können hergestellt werden
4-[4-(trans-4-Ethylcyclohexyl)phenyl]-2-fluorbenzylmethylether
4-[4-(trans-4-Vinylcyclohexyl)phenyl]-2-fluorbenzylmethylether
4-[4-(trans-4-(3-Butenyl)cyclohexyl)phenyl]-2-fluorbenzylmethylether
4-[4-(trans-4-Pentylcyclohexyl)phenyl]-2-fluorbenzylmethylether
4-[4-(trans-4-Ethylcyclohexyl)phenyl]-2,6-difluorbenzylmethylether
4-[4-(trans-4-Vinylcyclohexyl)phenyl]-2,6-difluorbenzylmethylether, Smp. (K/I): 76°C
4-[4-(trans-4-Propylcyclohexyl)phenyl]-2,6-difluorbenzylmethylether
4-[4-(trans-4-(3-Butenyl)cyclohexyl)phenyl]-2,6-difluorbenzylmethylether, Smp. (C/N): 62.8°C, Klp. (N/I): 73.3°C
4-[4-(trans-4-Pentylcyclohexyl)phenyl]-2,6-difluorbenzylmethylether
4-{4-[2-(trans-4-Propylcyclohexyl)ethyl]phenyl}-2-fluorbenzylmethylether
4-{4-[2-(trans-4-Pentylcyclohexyl)ethyl]phenyl}-2-fluorbenzylmethylether
4-{4-[2-(trans-4-Vinylcyclohexyl)ethyl]phenyl}-2-fluorbenzylmethylether
4-{4-[2-(trans-4-Propylcyclohexyl)ethyl]phenyl}-2,6-difluorbenzylmethylether
4-{4-[2-(trans-4-Pentylcyclohexyl)ethyl]phenyl}-2,6-difluorbenzylmethylether
4-{4-[2-(trans-4-Vinylcyclohexyl)ethyl]phenyl}-2,6-difluorbenzylmethylether
4-[4-(trans-5-Propyl-1,3-dioxan-2-yl)phenyl]-2-fluorbenzylmethylether
4-[4-(trans-5-Butyl-1,3-dioxan-2-yl)phenyl]-2-fluorbenzylmethylether
4-[4-(trans-5-Pentyl-1,3-dioxan-2-yl)phenyl]-2-fluorbenzylmethylether
4-[4-(trans-5-Vinyl-1,3-dioxan-2-yl)phenyl]-2-fluorbenzylmethylether
4-[4-(trans-5-(E-)Propenyl-1,3-dioxan-2-yl)phenyl]-2-fluorbenzylmethylether
4-[4-(trans-5-(3-Butenyl)-1,3-dioxan-2-yl)phenyl]-2-fluorbenzylmethylether
4-[4-(trans-5-Propyl-1,3-dioxan-2-yl)phenyl]-2,6-difluorbenzylmethylether
4-[4-(trans-5-Butyl-1,3-dioxan-2-yl)phenyl]-2,6-difluorbenzylmethylether
4-[4-(trans-5-Pentyl-1,3-dioxan-2-yl)phenyl]-2,6-difluorbenzylmethylether
4-[4-(trans-5-Vinyl-1,3-dioxan-2-yl)phenyl]-2,6-difluorbenzylmethylether
4-[4-(trans-5-(E-)Propenyl-1,3-dioxan-2-yl)phenyl]-2,6-difluorbenzylmethylether
4-[4-(trans-5-(3-Butenyl)-1,3-dioxan-2-yl)phenyl]-2,6-difluorbenzylmethylether
4-[5-(trans-4-Ethylcyclohexyl)pyridin-2-yl]-2-fluorbenzylmethylether
4-[5-(trans-4-Propylcyclohexyl)pyridin-2-yl]-2-fluorbenzylmethylether
4-[5-(trans-4-Pentylcyclohexyl)pyridin-2-yl]-2-fluorbenzylmethylether
4-[5-(trans-4-Ethylcyclohexyl)pyrimidin-2-yl]-2-fluorbenzylmethylether
4-[5-(trans-4-Propylcyclohexyl)pyrimidin-2-yl]-2-fluorbenzylmethylether
4-[5-(trans-4-Pentylcyclohexyl)pyrimidin-2-yl]-2-fluorbenzylmethylether
4-[4-(trans-4-Ethylcyclohexyl)phenyl]-2-fluorbenzylethylether
4-[4-(trans-4-Vinylcyclohexyl)phenyl]-2-fluorbenzylethylether
4-[4-(trans-4-Propylcyclohexyl)phenyl]-2-fluorbenzylethylether
4-[4-(trans-4-(3-Butenyl)cyclohexyl)phenyl]-2-fluorbenzylethylether
4-[4-(trans-4-Pentylcyclohexyl)phenyl]-2-fluorbenzylethylether
4-[4-(trans-4-Ethylcyclohexyl)phenyl]-2,6-difluorbenzylethylether
4-[4-(trans-4-Vinylcyclohexyl)phenyl]-2-fluorbenzylethylether
4-[4-(trans-4-Propylcyclohexyl)phenyl]-2,6-difluorbenzylethylether
4-[4-(trans-4-(3-Butenyl)cyclohexyl)phenyl]-2,6-difluorbenzylethylether
4-[4-(trans-4-Pentylcydohexyl)phenyl]-2,6-difluorbenzylethylether
4-[4-(trans-4-Ethylcyclohexyl)phenyl]-2-fluorbenzylethylether
4-[4-(trans-4-Vinylcyclohexyl)phenyl]-2-fluorbenzylethylether
4-[4-(trans-4-Propylcyclohexyl)phenyl]-2-fluorbenzylethylether
4-[4-(trans-4-(3-Butenyl)cyclohexyl)phenyl]-2-fluorbenzylethylether
4-[4-(trans-4-Pentylcyclohexyl)phenyl]-2-fluorbenzylethylether
4-[4-(trans-4-Ethylcyclohexyl)phenyl]-2,6-difluorbenzylethylether
4-[4-(trans-4-Vinylcyclohexyl)phenyl]-2-fluorbenzylethylether
4-[4-(trans-4-Propylcyclohexyl)phenyl]-2,6-difluorbenzylethylether
4-[4-(trans-4-(3-Butenyl)cyclohexyl)phenyl]-2,6-difluorbenzylethylether
4-[4-(trans-4-Pentylcyclohexyl)phenyl]-2,6-difluorbenzylethylether
4-[4-(trans-4-Allylcyclohexyl)phenyl]-2-fluorbenzylallylether
4-[4-(trans-4-Vinylcyclohexyl)phenyl]-2-fluorbenzylallylether
4-[4-(trans-4-Propylcyclohexyl)phenyl]-2-fluorbenzylallylether
4-[4-(trans-4-(3-Butenyl)cyclohexyl)phenyl]-2-fluorbenzylallylether
4-[4-(trans-4-Pentylcyclohexyl)phenyl]-2-fluorbenzylallylether
4-[4-(trans-4-Allylcyclohexyl)phenyl]-2,6-difluorbenzylallylether
4-[4-(trans-4-Vinylcyclohexyl)phenyl]-2-fluorbenzylallylether
4-[4-(trans-4-Propylcyclohexyl)phenyl]-2,6-difluorbenzylallylether
4-[4-(trans-4-(3-Butenyl)cyclohexyl)phenyl]-2,6-difluorbenzylallylether
4-[4-(trans-4-Pentylcyclohexyl)phenyl]-2,6-difluorbenzylallylether

### Beispiel 5

Zur Untersuchung der Eigenschaften der Verbindungen der Formel I in Gemischen wurden binäre Mischungen (BM) mit 4-(trans-4-Pentylcyclohexyl)benzonitril hergestellt. Die Schwellenspannung und die Ansprechzeiten wurden in einer TN-Zelle (low bias tilt) mit 8 um Plattenabstand bei 22°C gemessen; als Betriebsspannung wurde der 2,5-fache Wert der Schwellenspannung (V₁₀) gewählt. Die entsprechenden Daten für 4-(trans-4-Pentylcyclohexyl)benzonitril betragen: Klp. (N-I) = 54.6°C, V₁₀ = 1,62 V, tₒₙ = 22ms, t_{off} = 42 ms, Δn = 0,120.

| BM-1 | |
|---|---|
| 90 Gew.% | 4-(trans-4-Pentylcyclohexyl)benzonitril |
| 10 Gew.% | 4-[trans-4-(trans-4-Vinylcyclohexyl)cyclohexyl]-2-fluorbenzylmethylether |

Klp. (N/I): 59,3°C, V₁₀ = 1,67 V, tₒₙ = 29 ms, t_{off} = 41 ms, Δn = 0,123

| BM-2 | |
|---|---|
| 80 Gew.% | 4-(trans-4-Pentylcyclohexyl)benzonitril |
| 20 Gew.% | 4-[trans-4-(trans-4-Vinylcyclohexyl)cyclohexyl]-2-fluorbenzylmethylether |

Klp. (N/I): 63,8°C, V₁₀ = 1,73 V, tₒₙ= = 33 ms, t_{off} = 43 ms, Δn = 0,120

| BM-3 | |
|---|---|
| 90 Gew.% | 4-(trans-4-Pentylcyclohexyl)benzonitril |
| 10 Gew.% | 4-[trans-4-(trans-4-Vinylcydohexyl)cyclohexyl]-2,6-difluorbenzylmethylether |

Klp. (N/I): 55,5°C, V₁₀ = 1,58V, tₒₙ = 29 ms, t_{off} = 46 ms, Δn = 0,120

| BM-4 | |
|---|---|
| 80 Gew.% | 4-(trans-4-Pentylcyclohexyl)benzonitril |
| 20 Gew.% | 4-[trans-4-(trans-4-Vinylcyclohexyl)cyclohexyl]-2,6-difluorbenzylmethylether |

Klp. (N/I): 56,2°C, V₁₀ = 1,58 V, tₒₙ = 32 ms, t_{off} = 53 ms, Δn = 0,117

| BM-5 | |
|---|---|
| 90 Gew.% | 4-(trans-4-Pentylcyclohexyl)benzonitril |
| 10 Gew.% | 4-[trans-4-(trans-4-(3-Butenyl)cyclohexyl]-2-fluorbenzylmethylether |

Klp. (N/I): 61,5°C, V₁₀ = 1,69, tₒₙ = 24,7 ms, t_{off} = 40,8 ms. Δn = 0,1228

| BM-6 | |
|---|---|
| 80 Gew.% | 4-(trans-4-Pentylcyclohexyl)benzonitril |
| 20 Gew.% | 4-[trans-4-(trans-4-(3-Butenyl)cyclohexyl]-2-fluorbenzylmethylether |

Klp. (N/I): 68,4°C, V₁₀ =1,78, tₒₙ = 24,7 ms, t_{off}= 40,6 ms, Δn = 0,1196

## Patentansprüche

1. Verbindungen der allgemeinen Formel 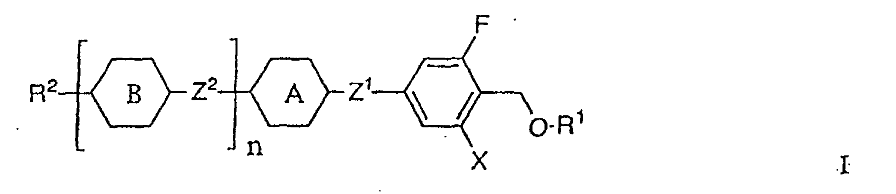 worin
R¹ C₁-C₅-Alkyl oder C₃-C₅-Alkenyl, worin auch eine CH₂-Gruppe durch ein Sauerstoffatom ersetzt sein kann;
X H oder F;
A und B unabhängig voneinander trans-1,4-Cyclohexylen, trans-1,3-Dioxan-2,5-diyl, Pyridin-2,5-diyl, Pyrimidin-2,5-diyl oder gegebenenfalls mit 1 oder 2 Fluoratomen substituiertes 1,4-Phenylen;
Z¹ eine Einfachbindung oder -CH₂-CH₂-
Z² unabhängig von Z eine Einfachbindung, -CH₂-CH₂-, -O-CH₂-, -CH₂-O-, -C≡C-, -(CH₂)₄-, -O(CH₂)₃-, -(CH₂)₃O-, -CH=CH-CH₂O- oder -CH=H-(CH₂)₂-, -OCH₂-CH=CH- oder -(CH₂)₂-CH=CH-; mit der Massgabe, dass mindestens eine der Gruppen Z¹ oder Z² eine Einfachbindung ist;
n 0 oder 1; und
R² C₂-C₁₂-Alkyl oder C₂-C₁₂-Alkenyl, worin auch eine oder mehrere nicht benachbarte CH₂-Gruppen durch Sauerstoff ersetzt und/oder eines oder mehrere Wasserstoffatome durch Fluoratome ersetzt sein können,
bedeuten, wobei R^{2'} dann nicht ein C₂-C₁₂-Alkyl darstellen darf, wenn R¹ ein C₁-C₅-Alkyl ist.

2. Verbindungen nach Anspruch 1, **dadurch gekennzeichnet, dass** der Rest R¹ Methyl, Ethyl oder Propyl bedeutet.

3. Verbindungen nach einem der Ansprüche 1 oder 2, **dadurch gekennzeichnet, dass** der Rest R² 2 bis 6 Kohlenstoffatome hat und Ethyl, Propyl, Butyl, Pentyl, Hexyl, Vinyl, 1E-Propenyl, 1E-Butenyl, 1E-Pentenyl, 1E-Hexenyl, 3-Butenyl oder 4-Pentenyl bedeutet.

4. Verbindungen nach einem der Ansprüche 1 bis 3, **dadurch gekennzeichnet, dass** Z² eine Einfachbindung oder -CH₂CH₂-bedeuten.

5. Verbindungen nach einem der Ansprüche 1 bis 4, **dadurch gekennzeichnet, dass** die Ringe A und B trans-1,4-Cyclohexylen, trans-1,3-Dioxan-2,5-diyl oder 1,4-Phenylen bedeuten.

6. Verbindungen der allgemeinen Formeln 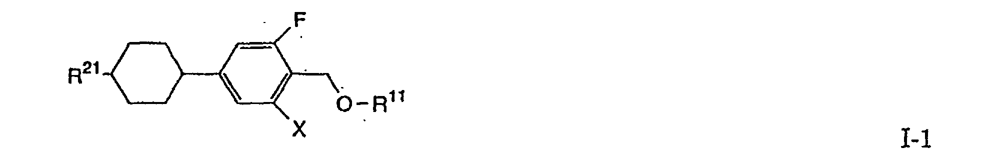 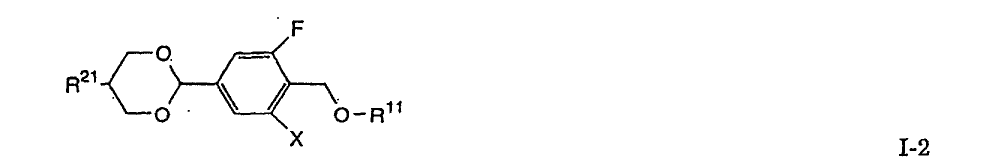 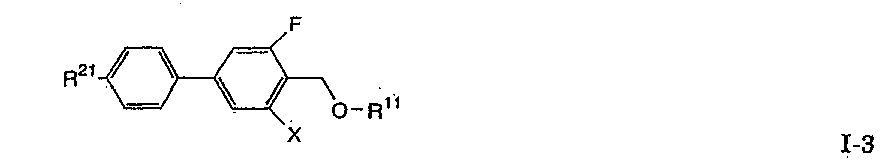 und 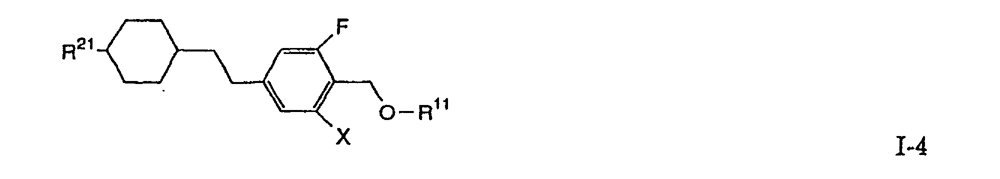 worin
R¹¹ C₁-C₃-Alkyl ;
X H oder F; und
R²¹ C₂-C₆-Alkyl oder C₂-C₆-Alkenyl bedeuten.

7. Verbindungen der allgemeinen. Formeln 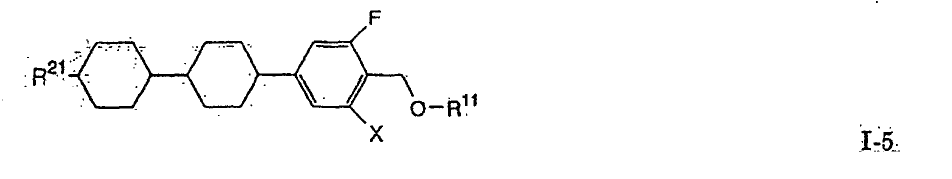 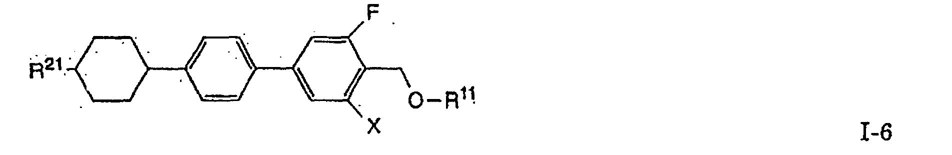 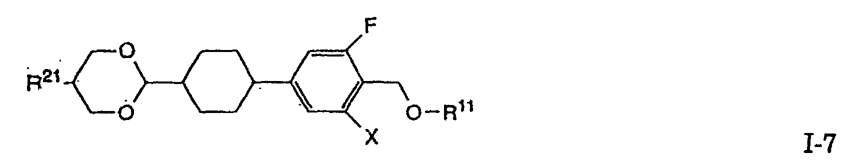 und 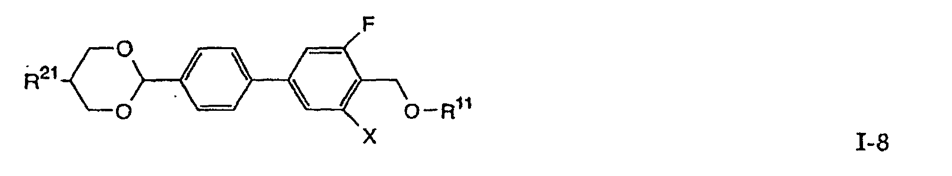 worin
R¹¹ C₁-C₃-Alkyl ;
X H oder F; und
R²¹ C₂-C₆-Alkyl oder C₂-C₆-Alkenyl bedeuten.

8. Verbindungen der allgemeinen Formeln 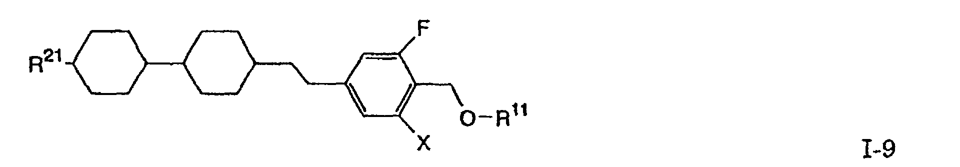 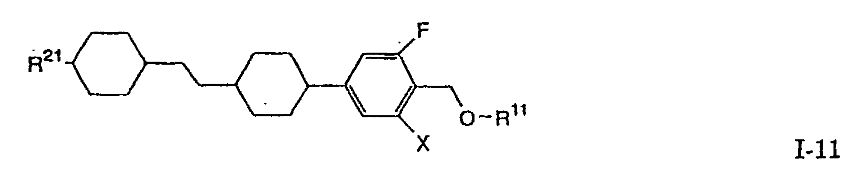 und 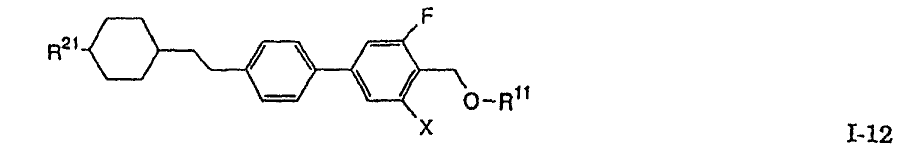 worin
R¹¹ C₁-C₃-Alkyl ;
X H oder F; und
R²¹ C₂-C₆-Alkyl oder C₂-C₆-Alkenyl bedeuten.

9. Flüssigkristallines Gemisch mit mindestens 2 Komponenten,
**dadurch gekennzeichnet, dass** mindestens eine Komponente eine Verbindung der in Anspruch 1 definierten Formel I ist.

10. Flüssigkristallines Gemisch nach Anspruch 9, **dadurch gekennzeichnet, dass** der Anteil an Verbindungen der Formel I 1-70 Gew.-% beträgt.

11. Flüssigkristallines Gemisch nach Anspruch 9 oder 10, **dadurch gekenzeichnet, dass** der Anteil an Verbindungen der Formel I 5-20 Gew.-% beträgt.

12. Verwendung der Verbindungen der in Anspruch 1 definierten Formel I für elektro-optische Vorrichtungen.

13. Verwendung von flüssigkristallinen Mischungen nach einem der Ansprüche 9 bis 11 für elektro-optische Vorrichtungen.

14. Elektro-optische Vorrichtung enthaltend eine Verbindung gemäss einem der Ansprüche 1-8 oder ein. flüssigkristallines Gemisch gemäss einem der Ansprüche 9-11.

## Claims

1. Compounds of the general formula 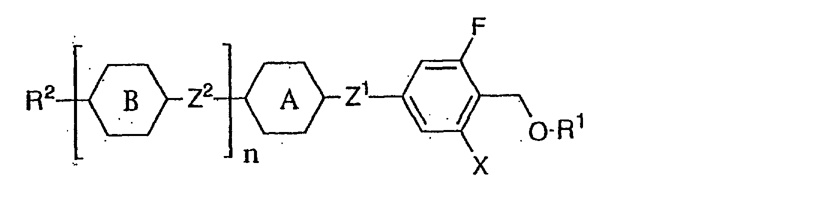 wherein
R¹ signifies C₁-C₅-alkyl or C₃-C₅-alkanyl in which also one CH₂ group can be replaced by an oxygen atom
X signifies H or F;
A and B each independently signify trans-1,4-cyclohexylene, trans-1,3-dioxane-2,5-diyl, pyridine-2,5-diyl, pyrimidine-2,5-diyl or 1,4-phenylene optionally substituted with 1 or 2 fluorine atoms;
Z¹ signifies a single bond or -CH₂-CH₂-,
Z² independent of Z¹ signifies a single bond, -CH₂-CH₂-, -O-CH₂-, -CH₂-O-, -C≡C-, -(CH₂)₄-, -O(CH₂)₃-, -(CH₂)₃O-, -CH=CH-CH₂O- or -CH=CH-(CH₂)₂-, -OCH₂-CH=CH- or -(CH₂)₂-CH=CH-; with the proviso that at least one of groups Z¹ and Z² is a single bond;
n signifies 0 or 1; and
R² signifies C₂-C₁₂-alkyl or C₂-C₁₂-alkenyl in which also one or more non-adjacent CH₂ groups can be replaced by oxygen and/or one or more hydrogen atoms can be replaced by fluorine atoms, wherein R² may not signify C₂-C₁₂-alkyl if R¹ signifies C₁-C₅-alkyl.

2. Compounds according to claim 1, wherein R¹ signifies methyl, ethyl or propyl.

3. Compounds according to claim 1 or claim 2, wherein R² has 2 to 6 carbon atoms and signifies ethyl, propyl, butyl, pentyl, hexyl, vinyl, 1E-propenyl, 1E-butenyl, 1E-pentenyl, 1E-hexenyl, 3-butenyl or 4-pentenyl.

4. Compounds according to any one of claims 1 to 3, wherein Z² signifies a single bond or -CH₂CH₂-.

5. Compounds according to any one of claims 1 to 4, wherein rings A and B signify trans-1,4-cyclohexylene, trans-1,3-dioxane-2,5-diyl or 1,4-phenylene.

6. Compounds of the general formulae 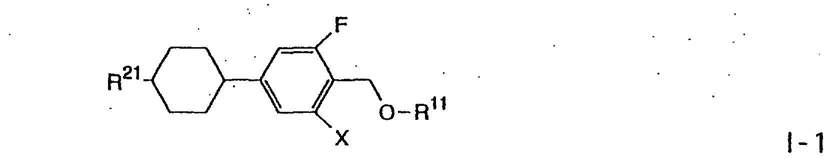 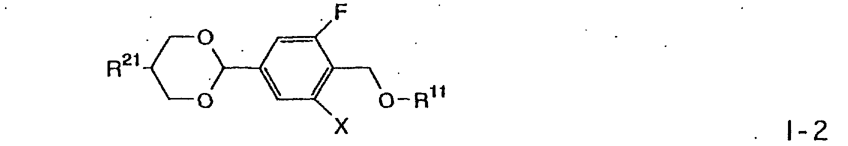 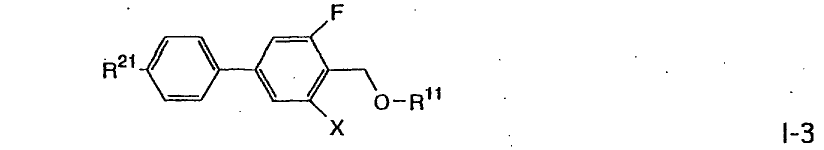 and 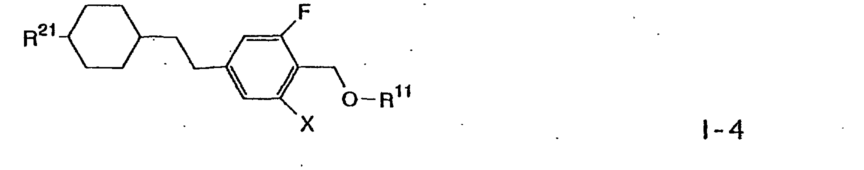 wherein
R¹¹ signifies C₁-C₃-alkyl;
X H or F; and
R²¹ signifies C₂-C₆-alkyl or C₂-C₆-alkenyl.

7. Compounds of the general formulae 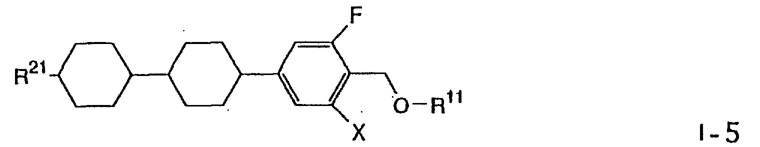 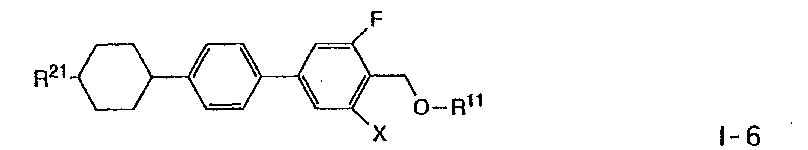 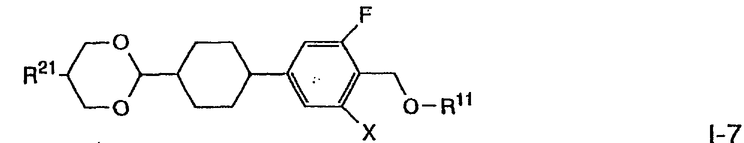 and 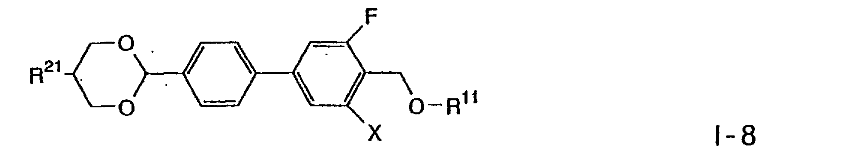 wherein
R¹¹ signifies C₁-C₃-alkyl;
X H or F; and
R²¹ signifies C₂-C₆-alkyl or C₂-C₆-alkenyl.

8. Compounds of the general formulae 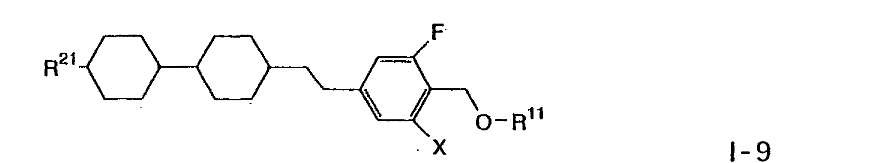 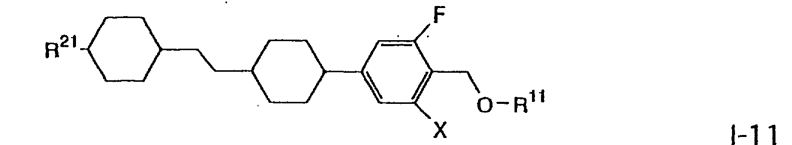 and 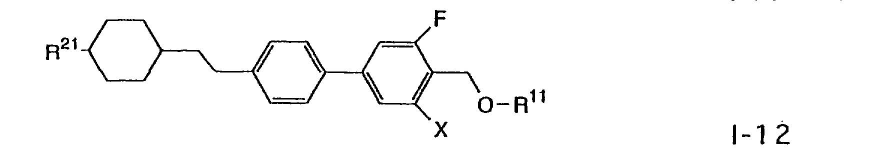 wherein
R¹¹ signifies C₁-C₃-alkyl;
X H or F; and
R²¹ signifies C₂-C₆-alkyl or C₂-C₆-alkenyl.

9. A liquid crystalline mixture having at least two components, wherein at least one component is a compound of formula I defined in claim 1.

10. A liquid crystalline mixture according to claim 9, wherein the content of compounds of formula I is 1-70 wt.%.

11. A liquid crystalline mixture according to claim 9 or 10, wherein the content of compounds of formula I is 5-20 wt.%.

12. The use of compounds of formula I defined in claim 1 for electro-optical devices.

13. The use of liquid crystalline mixtures according to any one of claims 9-11 for electro-optical devices.

14. Electro-optical device comprising a compound according to any one of claims 1-8 or a liquid crystalline mixture according to any one of claims 9-11.

## Revendications

1. Composés de formule générale, 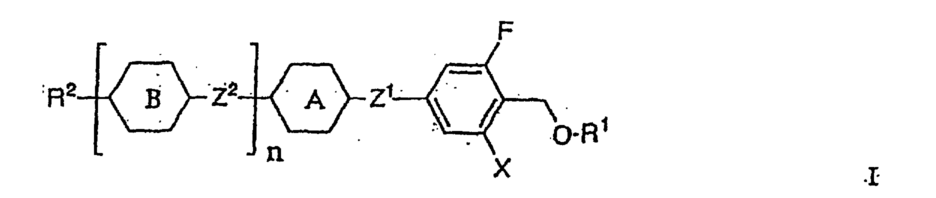 dans laquelle
R¹ représente un alkyle en C₁-C₅ ou alcényle en C₃-C₅, dans lequel également un groupe CH₂ peut être remplacé par un atome d'oxygène ;
X représente H ou F ;
A et B représentent indépendamment l'un de l'autre un trans-1,4-cyclohexylène, trans-1,3-dioxane-2,5-diyle, pyridine-2,5-diyle, pyrimidine-2,5-diyle ou 1,4-phénylène éventuellement substitué par 1 ou 2 atomes de fluor
Z¹ représente une liaison simple ou -CH₂-CH₂-
Z² représente indépendamment de Z¹ une liaison simple, -CH₂-CH₂-, -O-CH₂-, -CH₂-O-, -C≡C-, -(CH₂)₄-, -O(CH₂)₃-, -(CH₂)₃O-, -CH=CH-CH₂O- ou -CH=CH-(CH₂)₂-, -OCH₂-CH=CH- ou -(CH₂)₂-CH-CH- ; avec la condition qu'au moins un des groupes Z¹ ou Z² soit une liaison simple ;
n est 0 ou 1 ; et
R² représente un alkyle en C₂-C₁₂ ou alcényle en C₂-C₁₂, dans lequel également un ou plusieurs groupes CH₂ non voisins peuvent être remplacés par des atomes d'oxygène et/ou un ou plusieurs atomes d'hydrogène peuvent être remplacés par des atomes de fluor,
R² ne pouvant pas représenter un alkyle en C₂-C₁₂, lorsque R¹ est un alkyle en C₁-C₅.

2. Composés selon la revendication 1, **caractérisés en ce que** le groupe R¹ représente un méthyle, éthyle ou propyle.

3. Composés selon une des revendications 1 ou 2, **caractérisés en ce que** le groupe R² a de 2 à 6 atomes de carbone et représente un éthyle, propyle, butyle, pentyle, hexyle, vinyle, 1E-propényle, 1E-butényle, 1E-pentényle, 1E-hexényle, 3-butényle ou 4-pentényle.

4. Composés selon une des revendications 1 à 3, **caractérisés en ce que** Z² représente une.liaison simple ou -CH₂-CH₂-.

5. Composés selon une des revendications 1 à 4, **caractérisés en ce que** les cycles A et B représentent un trans-1,4-cyclohexylène, trans-1,3-dioxane-2,5-diyle ou 1,4-phénylène.

6. Composés des formules générales 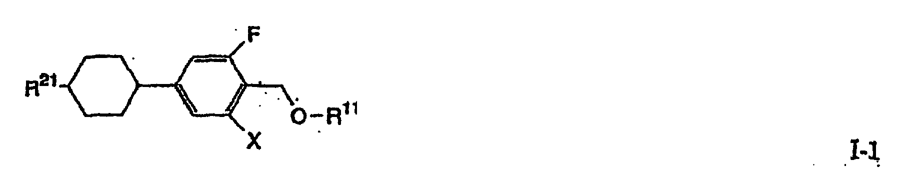 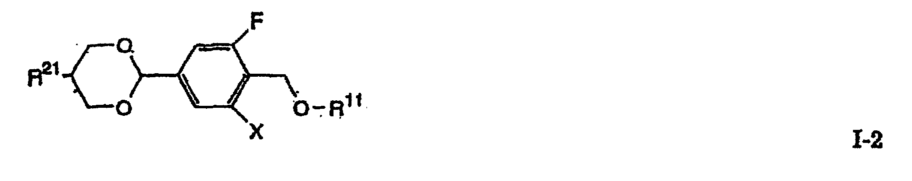 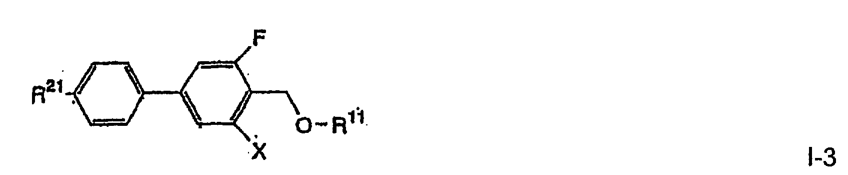 et 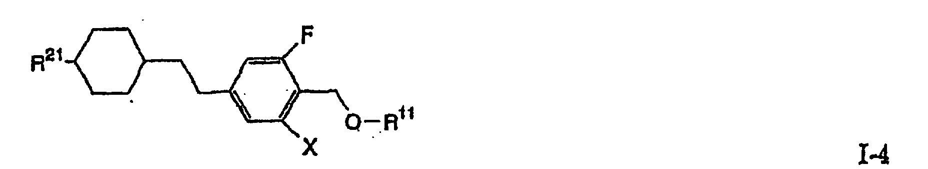 dans lesquelles
R¹¹ représente un alkyle en C₁-C₃;
X représente H ou F ; et
R²¹ représente un alkyle en C₂-C₆ ou alcényle en C₂-C₆.

7. Composés des formules générales 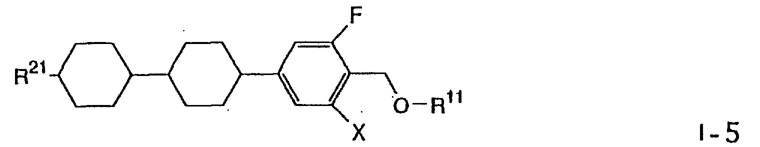 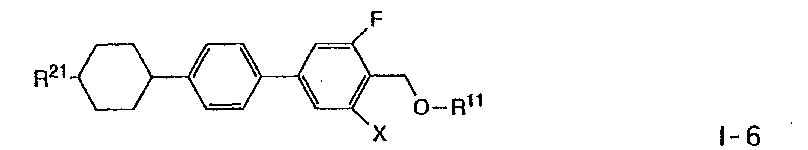 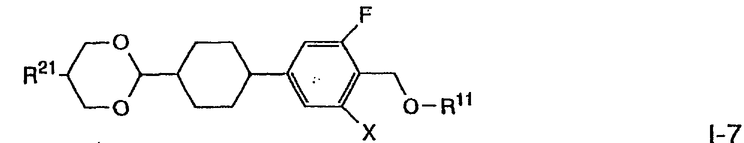 et 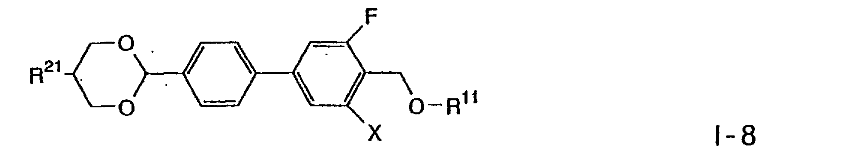 dans lesquelles
R¹¹ représente un alkyle en C₁-C₃ ;
X représente H ou F ; et
R²¹ représente un alkyle en C₂-C₆ ou alcényle en C₂-C₆.

8. Composés des formules générales 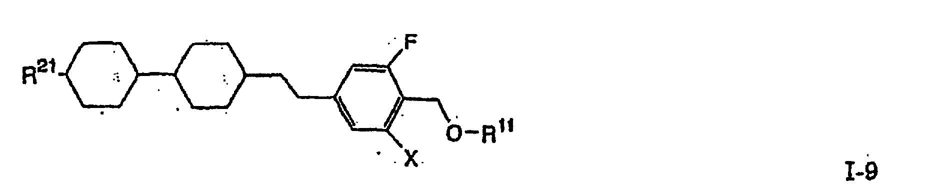 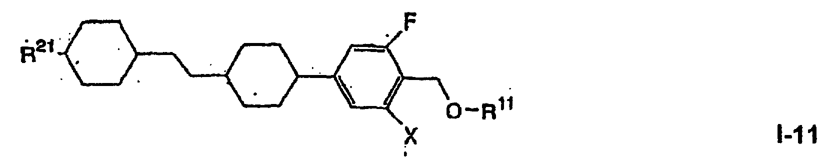 et 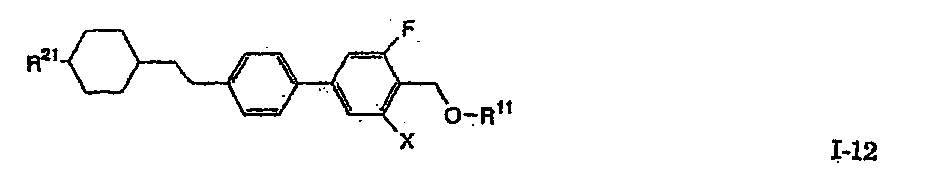 dans lesquelles
R¹¹ représente un alkyle en C₁-C₃ ;
X représente H ou F ; et
R²¹ représente un alkyle en C₂-C₆ ou alcényle en C₂-C₆.

9. Mélange pour cristaux liquides avec au moins 2 composants, **caractérisé en ce qu'**au moins un composant est un composé de formule I définie à la revendication 1.

10. Mélange pour cristaux liquides selon la revendication 9, **caractérisé en ce que** la proportion de composés de formule I varie de 1 à 70 % en poids.

11. Mélange pour cristaux liquides selon la revendication 9 ou 10, **caractérisé en ce que** la proportion de composés de formule I varie de 5 à 20 % en poids.

12. Utilisation des composés de formule I définie à la revendication 1 pour des dispositifs électro-optiques.

13. Utilisation de mélanges pour cristaux liquides selon une des revendications 9 à 11 pour des dispositifs électro-optiques.

14. Dispositif électro-optique contenant un composé selon une des revendications 1-8 ou un mélange pour cristaux liquides selon une des revendications 9-11.
